# EUROPEAN PATENT SPECIFICATION

(11) **EP 1 776 958 B1**
(45) Date of publication and mention of the grant of the patent: **17.12.2008**
(21) Application number: 06019172.3
(22) Date of filing: 09.06.2003
(51) Int. Cl.: A61K 38/00, A61K 38/17, A61P 25/28, A61P 9/10, A61P 27/16, A61P 1/18

(54) **Cell-permeable peptide inhibitors of the JNK signal transduction pathway**
Zell-durchlässige Peptidinhibitoren des JNK-Signaltransduktionsweges
Inhibiteurs peptidiques à permeabilité cellulaire, du mecanisme de transduction du signal de JNK

(30) Priority: 07.06.2002 US 165250
(43) Date of publication of application: 25.04.2007
(62) Divisional of application: 03740977.8
(73) Proprietor: Xigen S.A., 1003 Lausanne (CH)
(72) Inventor: Bonny, Christophe, 1006 Lausanne (CH)
(74) Representative: Graf von Stosch, Andreas

(56) References cited:
- WO-A-01/27268
- WO-A-94/04686
- WO-A-98/44106
- WO-A-98/49188
- BONNY C ET AL: "Cell-permeable peptide inhibitors of JNK: novel blockers of beta-cell death" DIABETES, NEW YORK, NY, US, vol. 50, no. 1, January 2001 (2001-01), pages 77-82, XP002172261 ISSN: 0012-1797
- VIVÈS E ET AL: "A truncated JIV-1 Tat protein basic domain rapidly translocates through the plasma embrane and accumulates in the cell nucleus" JOURNAL OF BIOLOGICAL CHEMISTRY, AMERICAN SOCIETY OF BIOLOGICAL CHEMISTS, BALTIMORE, MD, US, vol. 272, no. 25, 20 June 1997 (1997-06-20), pages 16010-16017, XP002172260 ISSN: 0021-9258
- BONNY C ET AL: "IB1, a JIP-1-related nuclear protein present in insulin-secreting cells" JOURNAL OF BIOLOGICAL CHEMISTRY, AMERICAN SOCIETY OF BIOLOGICAL CHEMISTS, BALTIMORE, MD, US, vol. 4, no. 273, 23 January 1998 (1998-01-23), pages 1843-1846, XP002076859 ISSN: 0021-9258
- LEE YONG HEE ET AL: "c-Jun N-terminal kinase (JNK) mediates feedback inhibition of the insulin signaling cascade." THE JOURNAL OF BIOLOGICAL CHEMISTRY. UNITED STATES 31 JAN 2003, vol. 278, no. 5, 31 January 2003 (2003-01-31), pages 2896-2902, XP002259338 ISSN: 0021-9258

## Description

### FIELD OF THE INVENTION

This invention relates generally to protein kinase inhibitors and more specifically to inhibitors of the protein kinase c-Jun amino terminal kinase.

### BACKGROUND OF THE INVENTION

The c-Jun amino terminal kinase (JNK) is a member of the stress-activated group of mitrogen-activated protein (MAP) kinases. These kinases have been implicated in the control of cell growth and differentiation, and, more generally, in the response of cells to environmental stimuli. The JNK signal transduction pathway is activated in response to environmental stress and by the engagement of several classes of cell surface receptors. These receptors can include cytokine receptors, serpentine receptors, and receptor tyrosine kinases. In mammalian cells, JNK has been implicated in such biological processes as oncogenic transformation and in mediating adaptive responses to environmental stress. JNK has also been associated with modulating immune responses, including maturation and differentiation of immune cells, as well effecting programmed cell death in cells identified for destruction by the immune system.

Several publications discuss JNK and the JNK signal transduction pathway. E.g. WO 98/49188 discloses inhibitors of the JNK signal transduction pathway and methods of use thereof. Particularly, WO 98/49188 identifies the JNK-interacting protein 1 (JIP-1), an inhibitor of the JNK1 protein. Furthermore, it discloses general methods for treating a pathological condition or methods of preventing the occurrence of a pathological condition in a patient by administration of a therapeutically effective amount of JIP-1 polypeptides. WO 98/49188 does not disclose the use of peptides, which contain either specific IB1 (JIP-1) or IB2 peptides and additional contain TAT sequences as transporter molecules for the treatment of hearing loss.

Similarly, WO 01/27268 discloses cell-permeable peptide inhibitors of the JNK signal transduction pathway, which bind to JNK proteins and inhibit JNK-mediated effects in JNK expressing cells. WO 01/27268 does not disclose the treatment of hearing loss. More particularly, WO 01/27268 does not disclose the use of peptides, which contain either specific IB1 or IB2 peptides and additional contain TAT sequences as transporter molecules for the treatment of hearing loss.

Bonny et al (Diabetes, Vol. 50, January 2001) describes peptide sequences related to JNKI-1. However, Bonny *et al.* (2001) is exclusively related to the IL-1 β induced apoptosis of pancreatic β cells and does not mention hearing loss or any disease related thereto.

Similarly, WO 98/44106 shows IB-1 and the use of IB-1-related polypeptides for the treatment of various diseases. WO 98/44106 does not specify the use of the specific IB1 or IB2 sequences according to the present invention for the treatment of hearing loss.

Vivès et al. (The Journal of Biological Chemistry, Vol. 272, No. 25, Issue of June 20, pp. 16010-16017, 1997), WO 94/04686, Bonny et al. (The Journal of Biological Chemistry, Vol. 273, No. 4, Issue of January 23, pp. 1843-1846, 1998), and Lee et al. (The Journal of Biological Chemistry, Vol. 278, No. 5, Issue of January 23, pp. 2896-2902, 2003) also discuss IB1- or JNK-related proteins. However, none of these documents discloses the treatment of hearing loss or even the use of peptides, which contain either specific IB1 or IB2 peptides and additional contain TAT sequences as transporter molecules for the treatment of hearing loss.

### SUMMARY OF THE INVENTION

The present invention is based in part on the discovery of peptides that are effective inhibitors of JNK proteins. The peptides, referred to herein as JNK peptide inhibitors, decrease the downstream cell-proliferative effects of c-Jun amino terminal kinase (JNK).

According, the invention includes the use of novel JNK inhibitor peptides ("JNKI peptides"), including any one of the amino acid sequences selected from SEQ ID NOs: 3-6 and 22, as well as chimeric peptides being any one of the amino acid sequences selected from SEQ ID NOs: 11-16 and 23-26 which include a JNK peptide inhibitor linked to a trafficking peptide that can be used to direct a peptide an which it is present do a desired cellular location or the use of nucleic acids encoding these peptides, in the manufacture of a medicament for the treatment or prevention of hearing loss in a-subject, wherein the peptide prevents damage to the hair cell stereccilia, hair cell apoptosis or neureral apoptosis. The trafficking sequence can be used to direct transport of the peptide across the plasma membrane. Alternatively, or in addition, the trafficking peptide can be used to direct the peptide to desired intracellular location, such as the nucleus.

The JNK inhibitor peptides can be present as polymers of L-amino acids. "Alternatively, the peptides can be present as polymers of D-amino acids.

The hearing loss may be a noise trauma. Thus, in one aspect, the peptide is to be administered before the subject is exposed to a noise trauma. In another aspect, the peptide is to be administered after the subject is exposed to a noise trauma. The noise trauma can be, e.g., at least 90 dB SPL. Alternatively, the hearing loss is caused by antibiotic treatment. Thus, in one aspect, the peptide is to be administered before the subject is exposed to an antibiotic. In another aspect, the peptide is to be administered after the subject is exposed to an antibiotic. The antibiotic is, e.g., an aminoglycoside.

The hearing loss may be caused by a chemotherapeutic agent. Thus, in one aspect, the peptide is to be administered before the subject is exposed to a chemotherapeutic agent In another aspect, the peptide is to be administered after the subject is exposed to a chemotherapeutic agent.

The administration of the peptides of the invention can be by any one administration route selected from: intrauricular, intraperitoneal, nasal, intravenous, oral and patch delivery.

Among the advantages provided by the invention is that the JNK inhibitor peptides used herein are small, and can be produced readily in bulk quantities and in high purity. The inhibitor peptides are also resistant to intracellular degradation, and are weakly immunogenic. Accordingly, the peptides are well suited for *in vitro* and *in vivo* applications in which inhibition of JNK-expression is desired.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In the case of conflict, the present specification, including definitions, will control. In addition, the materials, methods, and examples are illustrative only and not intended to be limiting.

Other features and advantages of the invention will be apparent from the following detailed description and claims.

### BRIEF DESCRIPTION OF THE DRAWINGS

FIGS. 1A-C are diagrams showing alignments of conserved JBD domain regions in the indicated transcription factors.
FIG. 2 is a diagram showing alignments of generic TAT-IB fusion peptides.
FIG. 3 is a histogram depicting inhibition of β-cell death by the minimal 23 amino acid long JBD domain of IB1 compared to the full 280 amino acid JBD domain.
FIG. 4 is an illustration demonstrating the effects of TAT, TAT-IB1 and TAT-IB2 peptides on phosphorylation of recombinant JNKs. Panel A shows inhibition of c-Jun, ATF2 and Elk1 phosphorylation by recombinant JNKs *in vitro.* Panel B shows dose response experiments similar to Panel A.
FIG. 5 is a histogram depicting L-TAT-IB inhibition ofphosphorylation by recombinant JNKs. Panel A shows L-TAT-IB inhibition of c-Jun, ATF2 and Elk1 phosphorylation by recombinant JNKs *in vitro* in the presence of MKK4. Panel B shows similar dose response experiments with MKK7.
FIG. 6 is an illustration demonstrating the inhibition of c-Jun phosphorylation by activated JNKs.

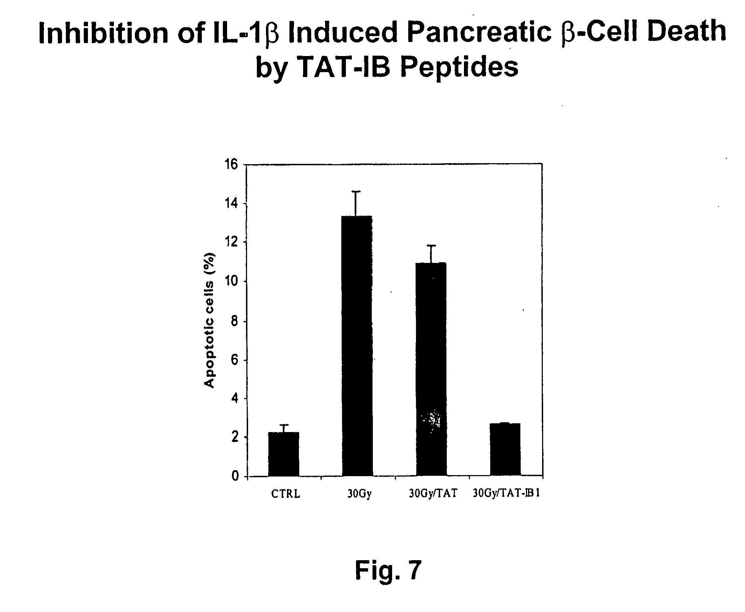

FIG. 7 is a histogram depicting short term inhibition of IL-1β induced pancreatic β-cell death by the I-TAT-IB peptides.

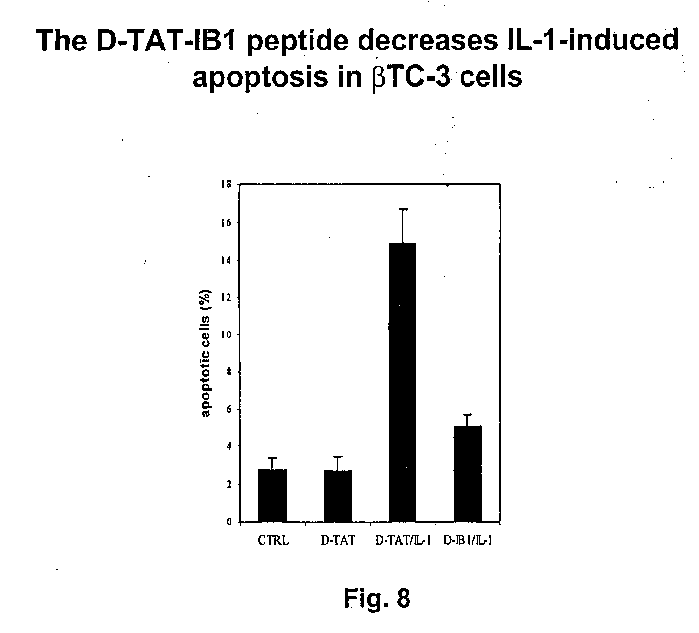

FIG. 8 is a histogram depicting short term inhibition of IL-1β induced pancreatic β-cell death by the D-TAT-IB peptides.
FIG. 9 is a histogram depicting long term inhibition or IL-1β induced pancreatic β-cell death by L-TAT-IB1 and D-TAT-IB1 peptides.
FIG.10 is a histogram depicting inhibition of irradiation induced human colon cancer WiDr cell death by L-TAT-IB1 and D-TAT-IB1 peptides.
FIG.11 is an illustration showing the modulation of JMK kinase activity by L-TAT, TAT-IB1 and D-TAT-IB1 peptides.
FIG.12 are graphs depicting the protective effects of the TAT-IB1 peptides in mice. Panel A shows the effect of irradiation on weight. Panel B shows the effect of irradiation on oedemus and erythemus status.
FIG.13 is a figure depicting the protective effect of D-JNK1 on noise-induced hearing loss. Panel A shows a schematic depiction of the experiment, panel B shows a graph of hearing loss, panels C and D depict histological examination of the contralateral (control) and D-JNK1-injected ear, respectively.
FIGs. 14 A and B are figures depicting the protective effect of D-JNK1 on antibiotic-induced hearing loss.
FIG.15 is a bar graph depicting the increased recovery of pancreatic islets subjected to D-JNK1 treatment during the isolation procedure.
FIG. 16A is an illustration demonstrating the sensitivity and specificity of the JNK-inhibitory (JNKI) peptides of the present invention against JNK activation and action. FIG.16A demonstrates the inhibitory effect of L-JNKI1 and D-JNKI1 on JNK activation and action in kinase assays with recombinant JNK1α1 and GST-Jun and GST-Elk1 substrates, respectively.
FIG. 16B is an illustration demonstrating the sensitivity and specificity of the JNK-inhibitory (JNKI) peptides of the present invention against JNK activation and action. FIG.16B demonstrates the inhibitory effect of the 20 amino add minimal JNK-inhibitory sequence of JIP-IB1 (L-form of JBD₂₀) in dose response experiments, using conditions similar to those in FIG. 16A and with decreasing amounts of L-JBD₂₀.
FIG. 16C is an illustration demonstrating the sensitivity and specificity of the JNK-inhibitory (JNKI) peptides of the present invention against JNK activation and action. FIG.16C demonstrates the specificity of the JNKI peptides of the present invention in blocking JNK activation using kinase assays with different recombinant kinases.
FIG.17A is an illustration demonstrating N-methyl-D-aspartate ("NMDA")-induced activation of JNK in untreated neurons (0) and in neurons exposed to 100 µM NMDA for 10 minutes (10') or for 30 minutes (30').
FIG.17B is an illustration that demonstrates the effects of the JNKI peptides of the present invention on the level of c-Jun phosphorylation and the amount of JNK after exposure to NMDA. In FIG. 17B, 4-fold more protein was loaded in the nuclear extracts (Nucl) than in the cytoplasmic ones (Cyt), and the abbreviations used are: C: Control; N: NMDA; L: L-JNI1+ NMDA; D: D-JNKI1 +NMDA.
FIG.17C is a histogram that depicts the quantification of c-fos expression by real-time PCR using extracted RNA. FIG. 17C illustrates the expression of c-fos relative to actin.
FIG.18 are illustrations and a histogram depicting the time course of NMDA neurotoxicity and neuroprotection by L-JNKI1, D-JNKI1 and two control peptides, TAT-empty (the TAT sequence alone, without JBD₂₀) and L-JNKI1-mut (wherein 6 amino acids have been mutated to alanine).
FIGS. 18A-18E are a series of micrographs that depict Hoechst-stained neurons at 24 hours after NMDA treatment.
FIG. 18F is a histogram depicting neuronal death at 12h, 24h, and 48h after NMDA exposure (100 µM NMDA), as indicated by LDH activity.
FIG.19 are illustrations and a histogram depicting transient ischemia in mice.
FIG.19A demonstrates the effect on infarct volume of a pretreatment in which an intracerebro-ventricular (icv) injection of D-JNKI1 (15.7 ng in 2 µL phosphate buffer solution (PBS)) was administered to a subject 1 hour prior to occlusion.
FIG. 19B demonstrates the effect on infarct volume where the icv injection of D-JKNI1 was administered 1 hour prior to occlusion or at 3 hours, 6 hours and 12 hours post-occlusion.
FIG. 20 are illustrations and a histogram demonstrating protection by D-JNKI1 against permanent focal ischemia in young rats (P14) that had been perfused 24 hours post-occlusion.
FIG. 20A is a series of illustrations that depicts examples of lesions from a control rat (left panel) and a rat treated with D-JNEI1 6 hours after occlusion (right panel).
FIG. 20B is a histogram depicting the infarct volumes, expressed as % of hemispheric volume, following the intra-peritoneal (i.p.) injection of D-JNKI1 at- 0.5 h before or at + 6 h or +12 h after occlusion.
FIG. 20C is a series of illustrations depicting the results of the immunohistochemistry for P-c-Jun in which c-Jun was phosphorylated in many neurons in the peri-infarcted cortex.

### DETAILED DESCRIPTION OF THE INVENTION

The present invention is based in part on the discovery of cell permeable peptides that inhibit the activated c-Jun amino terminal kinase (JNK) signaling pathway. These peptides are referred to herein as JNK inhibitor peptides.

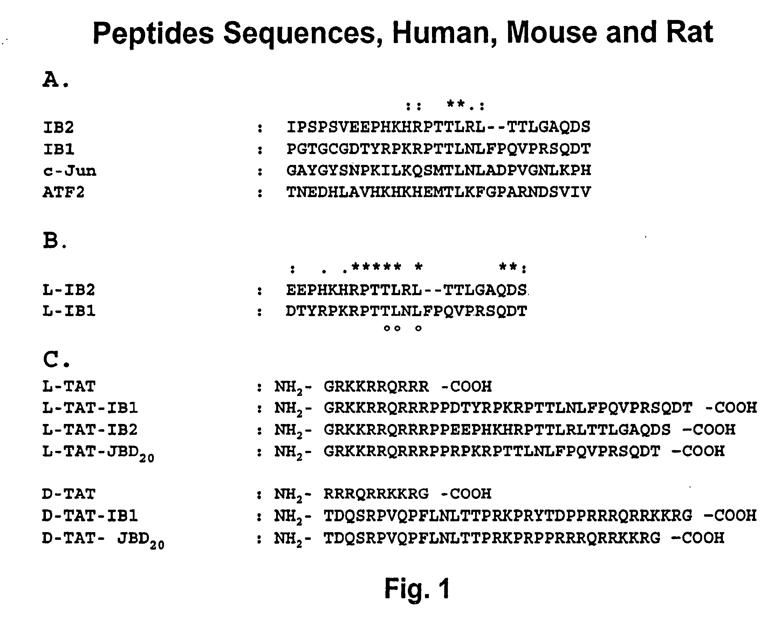

JNK inhibitor peptides were identified by inspecting sequence alignments between kJNK Binding Domains in various insulin binding (IB) proteins. The results of this alignment are shown in FIGS. 1A-1C. FIG. 1A depicts the region of highest homology between the JBDs of IB1, IB2, c-Jun and ATF2. Panel B depicts the amino acid sequence alignment of the JBDs of IB1 and IB2. Fully conserved residues are indicated by asterisks, while residues changed to Ala in the GFP-JBD_{23Mut} vector are indicated by open circles. FIG. 1C shows the amino acid sequences of chimeric proteins that include a JNK inhibitor peptide domain and a trafficking domain. In the example shown, the trafficking domain is derived from the human immunodeficiency virus (HIV) TAT polypeptide, and the JNK inhibitor peptide is derived from an IB1 polypeptide. Human, mouse, and rat sequences are identical in Panels B and C.

Sequence comparison between the JNK binding domains of IB1 [SEQ ID NO: 17], IB2 [SEQ ID NO: 18], c-Jun [SEQ ID NO: 19] and ATF2 [SEQ ID NO: 20] revealed a partially conserved 8 amino acid sequence (FIG. 1A). A comparison of the JBDs of IB1 and IB2 further revealed two blocks of seven and three amino acids that are highly conserved between the two sequences. These two blocks are contained within a peptide sequence of 23 amino acids in IB1 [SEQ ID NO: 1] and 21 amino acids in IB2 [SEQ ID NO: 2]. The 20 amino acid minimal JNK-inhibitory sequence of JIP-IB1 (L-form of JBD₂₀ (SEQ ID NO:21)) is shown in FIG. 1C.

The JNK inhibitor peptides as mentioned above can be used *in vitro* applications, *ex vivo*, and are suitable for *in vivo* applications. As JNKs and all its isoforms participate in the development and establishment of pathological states such as hearing loss or in pathways, the JNK peptides including any one of the amino acid sequences selected from SEQ ID NOs. 3-6 and 22, or being anyone of the amino acid sequences selected from SEQ ID NOs: 11-16 and 23-26 can be used to in the manufacture of a medicament for the treatment or prevention of hearing less in a subject

The JNK inhibitor peptides disclosed herein are presented in Table 1. The table presents the name of the JNK inhibitor peptide, as well as its sequence identifier number, length, and amino acid sequence.

**TABLE 1**

| **PEPTIDE NAME** | **SEQ ID** | **AA** | **Sequence** |
|---|---|---|---|
| L-IB1 | 1 | 23 | DTYRPKRPTT LNLFPQVPRS QDT |
| L-IB2 | 2 | 21 | EEPHKHRPTT LRLTTLGAQD S |
| D-IB1 | 3 | 23 | TDQSRPVQPF LNLTTPRKPR YTD |
| D-IB2 | 4 | 21 | SDQAGLTTLR LTTPRHKHPE E |
| L-IB (generic) | 5 | 19 | XRPTTLXLXX XXXXXQDS/TX |
| D-IB (generic) | 6 | 19 | XS/TDQXXXXXX XLXLTTPRX |
| L-TAT | 7 | 10 | GRKKRRQRRR |
| D-TAT | 8 | 10 | RRRQRRKKRG |
| L-generic-TAT | 9 | 17 | XXXXRKKRRQ RRRXXXX |
| D-generic-TAT | 10 | 17 | XXXXRRRQRR KKRXXXX |
| L-TAT-IB1 | 11 | 35 | GRKKRRQRRR PPDTYRPKRP TTLNLFPQVP RSQDT |
| L-TAT-IB2 | 12 | 33 | GRKKRRQRRR PPEEPHKHRP TTLRLTTLGA QDS |
| L-TAT-IB (generic) | 13 | 42 | XXXXXXXRKK RRQRRRXXXX XXXXRPTTLX LXXXXXXXQD S/TX |
| D-TAT-IB1 | 14 | 35 | TDQSRPVQPF LNLTTPRKPR YTDPPRRRQR RKKRG |
| D-TAT-IB2 | 15 | 33 | SDQAGLTTLR LTTPRHKHPE EPPRRRQRRK KRG |
| D-TAT-IB (generic) | 16 | 42 | XT/SDQXXXXXX XLXLTTPRXX XXXXXXRRRQ RRKKRXXXXX XX |
| EB1-long | 17 | 29 | PGTGCGDTYR PKRPTTLNLF PQVPRSQDT |
| IB2-long | 18 | 27 | IPSPSVEEPH KHRPTTLRLT TLGAQDS |
| c-Jun | 19 | 29 | GAYGYSNPKI LKQSMTLNLA DPVGNLKPH |
| ATF2 | 20 | 29 | TNEDHLAVHK HKHBMTLKFG PARNDSVIV |
| L-JBD₂₀ | 21 | 20 | RPKRPTTLNL FPQVPRSQDT |
| D-JBD₂₀ | 22 | 20 | TDQSRPVQPF LNLTTPRKPR |
| L-TAT-JNKI1 (*i*.*e*., L-TAT- JBD₂₀) | 23 | 32 | GRKKRRQRRR PPRPKRPTTL NLFPQVPRSQ DT |
| D-TAT-JNKI1 (*i*.*e*., D-TAT-JBD₂₀) | 24 | 32 | TDQSRPVQPF LNLTTPRRPR PPRRRQR RKKRG |
| L-TAT-JNKI1 (generic) | 25 | 34 | XXXXRKKRRQ RRRXXXXRPT TLXLXXXXXX XQDS/T |
| D-TAT-JNKI1 (generic) | 26 | 34 | S/TDQXXXXXXX LXLTTPRXXX XRRRQRRKKR XXXX |
| L-JBD₂₀-mut | 27 | 20 | RPKRPTAANA FPQVPRSQDT |
| 13.-JBD₂₀-mut | 28 | 20 | TDQSRPVAPF ANAATPRKPR |

### JNK INHIBITOR PEPTIDES

In one aspect, the invention provides use of a JNK inhibitor peptides including any one of the amino acid sequences selected from of SEQ ID NOs: 3-6 and 22, or a peptide being at least a 5% homologous to these peptide, in the manufacture of a medicament for the treatment or prevention of hearing loss in a subject, wherein the peptide prevents damage to the hair cells stereccilia, hair cell speptosis or neurenal speptosis. No particular length is implied by the term "peptide." In some embodiments, the JNK-inhibitor peptide is less than 280 amino acids in length, *e*.*g*., less than or equal to 150,100, 75, 50, 35, or 25 amino acids in length. In various embodiment, the JNK-binding inhibitor peptide includes the amino acid sequence of one or more of SEQ ID NOs: 3 and 22. In one embodiment, the JNK inhibitor peptides may bind JNK. In another embodiment the peptides may inhitibit the activation of at least one JNK activated transcription factor, *e*.*g*. c-Jun, ATF2 or Elk1.

Examples of JNK inhibitor peptides include a peptide which includes (in whole or in part) the sequence NH₂-DTYRPKRPTTLNLFPQVPRSQDT-_{COOH} [SEQ ID NO:1]. In another embodiment, the peptide includes the sequence NH₂-EEPHKHRPTTLRLTTLGAQDS-_{COOH} [SEQ ID NO:2] Alternatively, examples of JNK inhibitor peptides include a peptide which includes (in whole or in part) the sequence NH₂-RPKRPTTLNL FPQVPRSQDT-_{COOH} [SEQ ID NO:21].

The JNK inhibitor peptides can be polymers of L-amino acids, D-amino acids, or a combination of both. If the JNK inhibitor peptides are of D-amino acids, the peptides are D retro-inverso peptides. The term "retro-inverso isomer" refers to an isomer of a linear peptide in which the direction of the sequence is reversed, the term "D-retro-inverso isomer" refers to an isomer of a linear peptide in which the direction of the sequence is reversed and the chirality of each amino acid residue is inverted. *See*, *e*.*g*., Jameson et al., Nature, 368, 744-746 (1994); Brady et al., Nature, 368, 692-693 (1994). The net result of combining D-enantiomers and reverse synthesis is that the positions of carbonyl and amino groups in each amide bond are exchanged, while the position of the side-chain groups at each alpha carbon is preserved. Unless specifically stated otherwise, it is presumed that any given L-amino acid sequence of the invention may be made into an D retro-inverso peptide by synthesizing a reverse of the sequence for the corresponding native L-amino acid sequence.

For example, a D retro-inverso peptide has the sequence NH₂-TDQSRPVQPFLNLTTPRKPRYTD-_{COOH} [SEQ ID NO:3] or NH₂-SDQAGLTILRLTTPRHKHPEE-_{COOH} [SEQ ID NO: 4]. Alternatively, a D retro-inverso peptide includes the sequence NHᵣ- TDQSRPVQPF LNLTTPRKPR -COOH [SBQ ID NO:22]. It has been unexpectedly found that D-retro-inverso peptides have a variety of useful properties. For example, D-TAT and D-TAT-IB and D-TAT-JNKI peptides enter cells as efficiently as L-TAT and L-TAT-IB and D-TAT-JNKI peptides, and D-TAT and D-TAT-IB and D-TAT-JNKI peptides are more stable than the corresponding L-peptides. Further, while D-TAT-IB1 are -10-20 fold less efficient in inhibiting JNK than L-TAT-IB and L-TAT-JNKI, they are ∼50 fold more stable *in vivo*. Moreover, the D-retro-inverso JNKI peptides are protease-resistant. Finally, as is discussed further below, D-TAT-IB and D-TAT-JNKI peptides protect interleukin-1 treated and ionizing irradiated cells from apoptosis, and these peptides are useful in treating neurons, as the TAT sequence contains six pairs of amino acid that render the TAT sequence extremely sensitive to the neuronal proteases that are involved in peptide processing in the nervous system. *See e.g.,* Steiner et al., J. Biol. Chem. 267:23435 23438 (1992); Brugidou et al. Biochem. & Biophys. Res. Comm. 214:685-693 (1995).

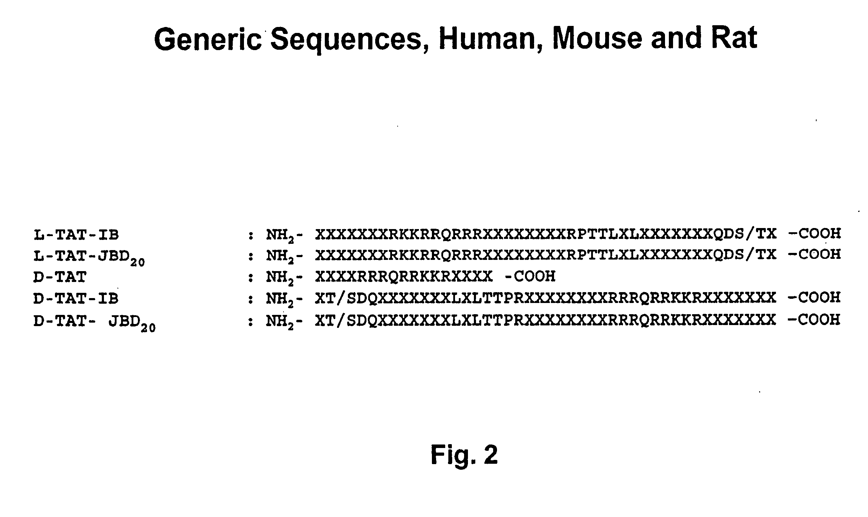

A JNK inhibitor peptide as used according to the invention includes the amino acid sequence NH₂-Xₙ-RPTTLXLXXXXXXXQDS/T -Xₙ-COOH [SEQ ID NO: 5, and residues 17-42 of L-TAT-IB, SEQ ID NO:13, as shown in FIG.2]. As used herein, Xₙ may be zero residues in length, or may be a contiguous stretch of peptide residues derived from SEQ ID NOS:1 and 21, preferably a stretch of between 1 and 7 amino acids in length, or may be 10, 20, 30 or more amino acids in length. The single residue represented by S/T may be either Ser or Thr in the generic sequence. In a further embodiment, a JNK inhibitor peptide as used herein may be a D retro-inverso peptide having the sequence NH₂-Xₙ-S/TDQXXXXXXXLXLTTPR-Xₙ-_{COOH} [SEQ ID NO: 6], and residues 17-42 of L-TAT-IB, SEQ ID NO:16, as shown in FIG.2].

JNK-inhibitor peptides are obtained or produced by methods well-known in the art, *e*.*g*. chemical synthesis, genetic engineering methods as discussed below. For example, a peptide corresponding to a portion of a JNK inhibitors peptide including a desired region or domain, or that mediates the desired activity *in vitro,* may be synthesized by use of a peptide synthesizer.

A candidate JNK inhibitor peptide is analyzed by hydrophilicity analysis (*see, e*.*g*., Hopp and Woods, 1981. Poc NatI Acad Sci USA 78: 3824-3828) that can be utilized to identify the hydrophobic and hydrophilic regions of the peptides, thus aiding in the design of substrates for experimental manipulation, such as in binding experiments, antibody synthesis. Secondary structural analysis may also be performed to identify regions of a JNK inhibitor peptide that assume specific structural motifs. *See e.g.*, Chou and Fasman, 1974. Biochem 13: 222-223. Manipulation, translation, secondary structure prediction, hydrophilicity and hydrophobicity profiles, open reading frame prediction and plotting, and determination of sequence homologies can be accomplished using computer software programs available in the art. Other methods of structural analysis including, *e.g.*, X-ray crystallography (*see, e.g.,* Engstrom, 1974. Biochem Exp Biol 11: 7-13)*;* mass spectroscopy and gas chromatography (*see. e.g,* METHODS IN PROTEIN SClENCE, 1997. J. Wiley and Sons, New York, NY) and computer modeling (*see*, *e.g*., Fletterick and Zoller, eds.,1986. Computer Graphics and Molecular Modeling, In: CURRENT COMMUNICATIONS IN MOLECULAR BIOLOGY, Cold Spring Harbor Laboratory Press, Cold Spring Harbour, NY) may also be employed.

The present invention additionally relates to nucleic acids that encode the inventive JNK-binding peptides having L-form amino acids, *e.g*., those L-peptides indicated in Table 1, as well as the complements of these sequences. Suitable sources of nucleic acids encoding JNK inhibitor peptides include the human IB1 nucleic acid (and the encoded protein sequences) available as GenBank Accession Nos. AF074091 and AAD20443, respectively. Other sources include rat IB1 nucleic acid and protein sequences are shown in GenBank Accession No. AF108959 and AAD22543, respectively. Human IB2 nucleic acid and protein sequences are shown in GenBank Accession No AF218778,

Nucleic acids encoding the JNK inhibitor peptides may be obtained by any method known in the art (*e.g*., by PCR amplification using synthetic primers hybridizable to the 3'-and 5'-termini of the sequence and/or by cloning from a cDNA or genomic library using an oligonucleotide sequence specific for the given gene sequence).

For recombinant expression of one or more JNK inhibitors peptides, the nucleic acid containing all or a portion of the nucleotide sequence encoding the peptide may be inserted into an appropriate expression vector (*i.e,* a vector that contains the necessary elements for the transcription and translation of the inserted peptide coding sequence). The regulatory elements may be heterologous (*i.e*, not the native gene promoter). Alternately, the necessary transcriptional and translational signals may also be supplied by the native promoter for the genes and/or their flanking regions.

A variety of host-vector systems may be utilized to express the peptide coding sequence(s). These include, but are not limited to: (i) mammalian cell systems that are infected with vaccinia virus, adenovirus, and the like; (ii) insect cell systems infected with baculovirus and the like; (iii) yeast containing yeast vectors or (iv) bacteria transformed with bacteriophage, DNA, plasmid DNA, or cosmid DNA. Depending upon the host-vector system utilized, any one of a number of suitable transcription and translation elements may be used.

Promoter/enhancer sequences within expression vectors may utilize plant, animal, insect, or fungus regulatory sequences, as defined in the invention. For example, promoter/enhancer elements can be used from yeast and other fungi (*e.g.*, the GAIA promoter, the alcohol dehydrogenase promoter, the phosphoglycerol kinase promoter, the alkaline phosphatase promoter). Alternatively, or in addition, they may include animal transcriptional control regions, *e.g*., (*i*) the insulin gene control region active within pancreatic β-cells (*see, e.g*., Hanahan, et al., 1985. Nature 315: 115-122); (*ii*) the immunoglobulin gene control region active within lymphoid cells *(see, e.g.,* Grosschedl, et al., 1984. Cell 38: 647-658); (*iii*) the albumin gene control region active within liver (*see, e.g*., Pinckert, et al., 1987. Genes and Dev 1: 268-276; (iv) the myelin basic protein gene control region active within brain oligodendrocyte cells (*see, e.g.,* Readhead, et al., 1987. Cell 48: 703-712); and (v) the gonadotropin-releasing hormone gene control region active within the hypothalamus (*see, e.g.,* Mason, et al., 1986. Science 234: 1372-1378), and the like.

Expression vectors or their derivatives include, *e.g*. human or animal viruses (*e.g.*, vaccinia virus or adenovirus); insect viruses (*e.g*., baculovirus); yeast vectors; bacteriophage vectors (*e.g*., lambda phage); plasmid vectors and cosmid vectors.

A host cell strain may be selected that modulates the expression of inserted sequences of interest, or modifies or processes expressed peptides encoded by the sequences in the specific manner desired. In addition, expression from certain promoters may be enhanced in the presence of certain inducers in a selected host strain; thus facilitating control of the expression of a genetically-engineered peptide Moreover, different host cells possess characteristic and specific mechanisms for the translational and post-translational processing and modification (*e.g*., glycosylation, phosphorylation, and the like) of expressed peptides. Appropriate cell lines or host systems may thus be chosen to ensure the desired modification and processing of the foreign peptide is achieved. For example, peptide expression within a bacterial system can be used to produce an unglycosylated core peptide; whereas expression within mammalian cells ensures "native" glycosylation of a heterologous peptide.

Also disclosed herein are derivatives, fragments, homologs, analogs and variants of inventive JNK inhibitor peptides as defined above and nucleic acids encoding these peptides. For nucleic acids, derivatives, fragments, and analogs provided herein are defined as sequences of at least 6 (contiguous) nucleic acids, and which have a length sufficient to allow for specific hybridization. For amino acids, derivatives, fragments, and analogs provided herein are defined as sequences of at least 4 (contiguous) amino acids, a length sufficient to allow for specific recognition of an epitope.

The length of the fragments are less than the length of the corresponding full-length nucleic acid or polypeptide from which the JNK inhibitor peptide, or nucleic acid encoding same, is derived. Derivatives and analogs may be full length or other than full length, if the derivative or analog contains a modified nucleic acid or amino acid. Derivatives or analogs of the JNK inhibitor peptides include, *e.g.*, molecules including regions that are substantially homologous to the peptides, in various embodiments, by at least about 95%, 98%, or even 99%, identity over an amino acid sequence of identical size or when compared to an aligned sequence in which the alignment is done by a computer homology program known in the art For example sequence identity can be measured using sequence analysis software (Sequence Analysis Software Package of the Genetics Computer Group, University of Wisconsin Biotechnology Center. 1710 University Avenue, Madison, Wis. 53705), with the default parameters therein.

In the case of polypeptide sequences, which are less than 100% identical to a reference sequence, the non-identical positions are preferably, but not necessarily, conservative substitutions for the reference sequence. Conservative substitutions typically include substitutions within the following groups: glycine and alanine; value, isoleucine, and leucine; aspartic acid and glutamic acid; asparagine and glutamine; serine and threonine; lysine and arginine; and phenylalanine and tyrosine. Thus, included in the invention are peptides having mutated sequences such that they remain homologous, *e.g.* in sequence, in function, and in antigenic character or other function, with a protein having the corresponding parent sequence. Such mutations can, for example, be mutations involving conservative amino acid changes, *e.g.*, changes between amino acids of broadly similar molecular properties. For example, interchanges within the aliphatic group alanine, valine, leucine and isoleucine can be considered as conservative. Sometimes substitution of glycine for one of these can also be considered conservative. Other conservative interchanges include those within the aliphatic group aspartate and glutamate; within the amide group asparagine and glutamine; within the hydroxyl group serine and threonine; within the aromatic group phenylalanine, tyrosine and tryptophan; within the basic group lysine, arginine and histidine; and within the sulfur-containing group methionine and cysteine. Sometimes substitution within the group methionine and leucine can also be considered conservative. Preferred conservative substitution groups are aspartate-glutamate; asparagine-glutamine; valino-leucine-isoleucine; alanine-valine; phenylalanine- tyrosine; and lysine-arginine.

Where a particular polypeptide is said to have a specific percent identity to a reference polypeptide of a defined length, the percent identity is relative to the reference peptide. Thus, a peptide that is 50% identical to a reference polypeptide that is 100 amino acids long can be a 50 amino acid polypeptide that is completely identical to a 50 amino acid long portion of the reference polypeptide. It might also be a 100 amino acid long polypeptide, which is 50% identical to the reference polypeptide over its entire length. Of course, other polypeptides will meet the same criteria.

The invention also encompasses allelic variants of the disclosed polynucleotides or peptides; that is, naturally-occurring alternative forms of the isolated polynucleotide that also encode peptides that are identical, homologous or related to that encoded by the polynucleotides. Alternatively, non-naturally occurring variants may be produced by mutagenesis techniques or by direct synthesis.

Species homologs of the disclosed polynucleotides and peptides are also provided by the present invention. "Variant" refers to a polynucleotide or polypeptide differing from the polynucleotide or polypeptide of the present invention, but retaining essential properties thereof. Generally, variants are overall closely similar, and in many regions, identical to the polynucleotide or polypeptide of the present invention. The variants may contain alterations in the coding regions, non-coding regions, or both.

In some embodiments, altered sequences include insertions such that the overall amino acid sequence is lengthened while the protein retains trafficking properties. Additionally, altered sequences may include random or designed internal deletions that shorten the overall amino acid sequence while the protein retains transport properties.

The altered sequences can additionally or alternatively be encoded by polynucleotides that hybridize under stringent conditions with the appropriate strand of the naturally-occurring polynucleotide encoding a polypeptide or peptide from which the JNK inhibitor peptide is derived. The variant peptide can be tested for JNK-binding and modulation of JNK-mediated activity using the herein described assays. 'Stringent conditions' are sequence dependent and will be different in different circumstances. Generally, stringent conditions can be selected to be about 5°C lower than the thermal melting point (T_{M}) for the specific sequence at a defined ionic strength and pH. The T_{M} is the temperature (under defined ionic strength and pH) at which 50% of the target sequence hybridizes to a perfectly matched probe. Typically, stringent conditions will be those in which the salt concentration is at least about 0.02 molar at pH 7 and the temperature is at least about 60°C. As other factors may affect the stringency of hybridization (including, among others, base composition and size of the complementary strands), the presence of organic solvents and the extent of base mismatching, the combination of parameters is more important than the absolute measure of any one.

High stringency can include, *e.g.*, Step 1: Filters containing DNA are pretreated for 8 hours to overnight at 65°C in buffer composed of 6X SSC, 50 mM Tris-HCl (pH 7.5), 1 mM EDTA, 0.02% PVP, 0.02% Ficoll, 0.02% BSA, and 500 µg/ml denatured salmon sperm DNA. Step 2: Filters are hybridized for 48 hours at 65°C in the above prehybridization mixture to which is added 100 mg/ml denatured salmon sperm DNA and 5-20 x 10⁶ cpm of ³²P-labeled probe. Step 3: Filters are washed for 1 hour at 37°C in a solution containing 2X SSC, 0.01 % PVP, 0.01% Ficoll, and 0.01% BSA. This is followed by a wash in 0.1X SSC at 50°C for 45 minutes. Step 4: Filters are autoradiographed. Other conditions of high stringency that may be used are well known in the art. *See, e.g.,* Ausubel et al., (eds.), 1993, CURRENT PROTOCOLS IN MOLECULAR BIOLOGY, John Wiley and Sons, NY; and Kriegler, 1990, GENE TRANSFER AND EXPRESSION, A LABORATORY MANUAL, Stockton Press, NY.

Moderate stringency conditions can include the following: Step 1: Filters containing DNA are pretreated for 6 hours at 55°C in a solution containing 6X SSC, 5X Denhardt's solution, 0.5% SDS and 100 mg/ml denatured salmon sperm DNA. Step 2: Filters are hybridized for 18-20 hours at 55°C in the same solution with 5-20 x 106 cpm ³²P-labeled probe added. Step 3: Filters are washed at 37°C for 1 hour in a solution containing 2X SSC, 0.1% SDS, then washed twice for 30 minutes at 60°C in a solution containing 1X SSC and 0.1% SDS. Step 4: Filters are blotted dry and exposed for autoradiography. Other conditions of moderate stringency that may be used are well-known in the art *See, e.g.,* Ausubel et al., (eds.),1993, CURRENT PROTOCOLS IN MOLECULAR BIOLOGY, John Wiley and Sons, NY; and Kriegler,1990, GENE TRANSFER AND EXPRESSION, A LABORATORY MANUAL, Stockton Press, NY.

Low stringency can include: Step 1: Filters containing DNA are pretreated for 6 hours at 40°C in a solution containing 35% formamide, 5X SSC, 50 mM Tris-HCl (pH 7.5), 5 mM EDTA, 0.1% PVP, 0.1% Ficoll,1% BSA, and 500 µg/ml denatured salmon sperm DNA. Step 2: Filters are hybridized for 18-20 hours at 40°C in the same solution with the addition of 0.02% PVP, 0.02% Ficoll, 0.2% BSA, 100 µg(ml salmon sperm DNA, 10% (wt/vol) dextran sulfate, and 5-20 x 106 cpm ³²P-labeled probe. Step 3: Filters are washed for 1.5 hours at 55°C in a solution containing 2X SSC, 25 mM Tris-HCl (pH 7.4), 5 mM EDTA, and 0.1% SDS. The wash solution is replaced with fresh solution and incubated an additional 1.5 hours at 60°C. Step 4: Filters are blotted dry and exposed for autoradiography. If necessary, filters are washed for a third time at 65-68°C and reexposed to film. Other conditions of low stringency that may be used are well known in the art (*e.g.,* as employed for cross-species hybridizations). *See, e.g.,* Ausubel et al., (eds.), 1993, CURRENT PROTOCOLS IN MOLECULAR BIOLOGY, John Wiley and Sons, NY; and Kriegler, 1990, GENE TRANSFER AND EXPRESSION, A LABORATORY MANUAL, Stockton Press, NY.

### Chimeric Peptides Including a JNK Inhibitor Domains and a Trafficking Domain

In another aspect the invention provides use of chimeric peptides being any one of the amino acid sequences selected from SEQ IDs 11-16 and 23-26, or a peptide being at least about 95% homologous to these peptides, as defined above, which include a first and second domain for the treatment or prevention of hearing less in a subject, wherein the peptide prevents damage to the hair cells stereocilia, hair cell apoptosis or neuronal apoptosis. The first domain includes a tracking sequence, while the second domain includes a JNK inhibitor sequence linked by a covalent bond, *e.g.* peptide bond, to the first domain. The first and second domains can occur in any order in the peptide, and the peptide can include one or more of each domain.

A trafficking sequence is a sequence of amino acids that directs a peptide in which it is present to a desired cellular destination. Thus, the trafficking sequence can direct the peptide across the plasma membrane, *e.g.*, from outside the cell, through the plasma membrane, and into the cytoplasm. Alternatively, or in addition, the trafficking sequence can direct the peptide to a desired location within the cell, *e.g*., the nucleus, the ribosome, the ER, a lysosome, or peroxisome.

In some embodiments, the trafficking peptide is derived from a known membrane-translocating sequence. For example, the trafficking peptide may include sequences from the human immunodeficiency virus (HTV) 1 TAT protein. This protein is described in, *e.g.*, U.S. Patent Nos. 5,804,604 and 5,674,980. The JNK inhibitor peptide is linked to some of the entire 86 amino acids that make up the TAT protein. Herein, a functionally effective fragment or portion of a TAT protein that has fewer than 86 amino acids, which exhibits uptake into cells, and optionally uptake into the cell nucleus, can be used. *See e.g.*, Vives et al., J. Biol. Chem., 272(25):16010-17 (1997). In one embodiment, the fragment includes a peptide containing TAT residues 48 -57, *e.g.* NH₃-GRKKRRQRRR-_{COOH}[SEQ ID NO:7] or a generic TAT sequence NH₃-Xₙ-RKKRRQRRR-Xₙ-_{COOH} [SEQ ID NO: 9]. A TAT peptide that includes the region that mediates entry and uptake into cells can be defined using known techniques*. See, e.g.*, Franked et al., Proc. Natl. Acad. Sci, USA 86: 7397-7401 (1989).

The TAT sequence may be linked either to the N-terminal or the C-terminal end of JNK inhibitor sequence. A hinge of two proline residues may be added between the TAT and JNK inhibitor peptide to create the full fusion peptide. For example, L-amino acid fusion peptides used here in may be the L-TAT-IB1 peptide [SEQ ID NO:11], the L-TAT-IB2 peptide [SEQ ID NO:12], or the generic L-TAT-IB peptide [SEQ ID NO:13]. Alternatively, L-amino acid fusion peptides may be generic L-TAT-JNKI peptide [SEQ ID NO:25]. D retro-inverso fusion peptides may be the D-TAT-IB1 peptide [SEQ ID NO:14], the D-TAT-IB2 peptide [SEQ ID NO:15], or the generic D-TAT-IB peptide [SEQ ID NO:16]. Alternatively, D retro-inverso fusion peptides may include the D-TAT-JNKI peptide [SEQ ID NO:22] or the generic D-TAT-JNKI peptide [SEQ ID NO:26]. The TAT peptide may be a D retro-inverso peptide having the sequence NH₂-Xₙ-RRRQRRKKR-Xₙ-_{COOH} [SEQ ID NO:10]. In SEQ ID NOs:5-6, 9-10, 13, 16, and 25-26, the number of "X" residues is not limited to the one depicted and can equal any number of amino acid residues, including zero, and may vary as described above.

The trafficking sequence can be a single (*i.e*., continuous) amino acid sequence present in the TAT sequence. Alternatively it can be two or more amino acid sequences, which are present in TAT protein, but in the naturally-occurring protein are separated by other amino acid sequences. As used herein, TAT protein includes a naturally-occurring amino acid sequence that is the same as that of naturally-occurring TAT protein, or its functional equivalent protein or functionally equivalent fragments thereof (peptides). Such functional equivalent proteins or functionally equivalent fragments possess uptake activity into the cell and into the cell nucleus that is substantially similar to that of naturally-occurring TAT protein. TAT protein can be obtained from naturally-occurring sources or can be produced using genetic engineering techniques or chemical synthesis.

The amino acid sequence of naturally-occurring HIV TAT protein can be modified, for example, by addition, deletion and/or substitution of at least one amino acid present in the naturally-occurring TAT protein, to produce modified TAT protein (also referred to herein as TAT protein). Modified TAT protein or TAT peptide analogs with increased or decreased stability can be produced using known techniques. In some embodiments TAT proteins or peptides include amino acid sequences that are substantially similar, although not identical, to that of naturally-occurring TAT protein or portions thereof. In addition, cholesterol or other lipid derivatives can be added to TAT protein to produce a modified TAT having increased membrane solubility.

Variants of the TAT protein can be designed to modulate intracellular localization of TAT- JNK inhibitor peptide. When to be added exogenously, such variants are usually designed such that the ability of TAT to enter cells is retained (*i.e*., the uptake of the variant TAT protein or peptide into the cell is substantially similar to that of naturally-occurring HIV TAT). For example, alteration of the basic region thought to be important for nuclear localization (*see, e.g.,* Dang and Lee, J. Biol. Chem. 264:18019-18023 (1989); Hauber et al., J. Virol. 63:1181-1187 (1989); Ruben et al., J. Virol. 63:1-8 (1989)) can result in a cytoplasmic location or partially cytoplasmic location of TAT, and therefore, of the JNK inhibitor peptide. Alternatively, a sequence for binding a cytoplasmic or any other component or compartment (*e.g.*, endoplasmic reticule, mitochondria, gloom apparatus, lysosomal vesicles,) can be introduced into TAT in order to retain TAT and the JNK inhibitor peptide in the cytoplasm or any other compartment to confer regulation upon uptake of TAT and the JNK inhibitor peptide.

Other sources for the trafficking peptide include, *e.g.*, VP22 (described in, *e.g.,* WO 97/05265; Elliott and O'Hare, Cell 88: 223-233 (1997)), or non-viral proteins (Jackson et al, Proc. Natl. Acad. Sci. USA 89: 10691-10695 (1992)).

The JNK inhibitor sequence and the trafficking sequence can be linked by chemical coupling in any suitable manner known in the art. Many known chemical cross-linking methods are non-specific, *i.e.*; they do not direct the point of coupling to any particular site on the transport polypeptide or cargo macromolecule. As a result, use of non-specific cross-linking agents may attack functional sites or sterically block active sites, rendering the conjugated proteins biologically inactive.

One way to increasing coupling specificity is to directly chemical coupling to a functional group found only once or a few times in one or both of the polypeptides to be cross-linked. For example, in many proteins, cysteine, which is the only protein amino acid containing a thiol group, occurs only a few times. Also, for example, if a polypeptide contains no lysine residues, a cross-linking reagent specific for primary amines will be selective for the amino terminus of that polypeptide. Successful utilization of this approach to increase coupling specificity requires that the polypeptide have the suitably rare and reactive residues in areas of the molecule that may be altered without loss of the molecule's biological activity.

Cysteine residues may be replaced when they occur in parts of a polypeptide sequence where their participation in a cross-linking reaction would otherwise likely interfere with biological activity. When a cysteine residue is replaced, it is typically desirable to minimize resulting changes in polypeptide folding. Changes in polypeptide folding are minimized when the replacement is chemically and sterically similar to cysteine. For these reasons, serine is preferred as a replacement for cysteine. As demonstrated in the examples below, a cysteine residue may be introduced into a polypeptide's amino acid sequence for cross-linking purposes. When a cysteine residue is introduced, introduction at or near the amino or carboxy terminus is preferred. Conventional methods are available for such amino acid sequence modifications, whether the polypeptide of interest is produced by chemical synthesis or expression of recombinant DNA

Coupling of the two constituents can be accomplished via a coupling or conjugating agent. There are several intermolecular cross-linking reagents which can be utilized, See *for example,* Means and Feeney, CHEMICAL MODIFICATION OF PROTEINS, Holden-Day, 1974, pp. 39-43. Among these reagents are, for example, J-succinimidyl 3-(2-pyridyldithio) propionate (SPDP) or N, N- (1,3-phenylene) bismaleimide (both of which are highly specific for sulfhydryl groups and form irreversible linkages); N, N-ethylene-bis- (iodoacetamide) or other such reagent having 6 to 11 carbon methylene bridges (which relatively specific for sulfhydryl groups); and 1,5-difluoro-2,4-dinitrobenzene (which forms irreversible linkages with amino and tyrosine groups). Other cross-linking reagents useful for this purpose include: p,p'-difluoro-m,m'-dinitrodiphenylsulfone (which forms irreversible cross-linkages with amino and phenolic groups); dimethyl adipimidate (which is specific for amino groups); phenol-1,4-disulfonylchloride (which reacts principally with amino groups); hexamethylenediisocyanate or diisothiocyanate, or azophenyl-p-diisocyanate (which reacts principally with amino groups); glutaraldehyde (which reacts with several different side chains) and disdiazobenzidine (which reacts primarily with tyrosine and histidine).

Cross-linking reagents may be homobifunctional, *i.e.*, having two functional groups that undergo the same reaction. A preferred homobifunctional cross-linking reagent is bismaleimidohexane ("BMH"). BMH contains two maleimide functional groups, which react specifically with sulfhydryl-containing compounds under mild conditions (pH 6.5-7.7). The two maleimide groups are connected by a hydrocarbon chain. Therefore, BMH is useful for irreversible cross-linking of polypeptides that contain cysteine residues.

Cross-linking reagents may also be heterobifuncdonal. Heterobifunctional cross-linking agents have two different functional groups, for example an amine-reactive group and a thiol-reactive group, that will cross-link two proteins having free amines and thiols, respectively. Examples of heterobifunctional cross-linking agents are succinimidyl 4-(N maleimidomethyl) cyclohexane-1-carboxylate ("SMCC"), m-maleimidobenzoyi-N-hydroxysuccinimide ester ("MBS"), and succinimide 4-(p-maleimidophenyl) butyrate ("SMPB"), an extended chain analog of MBS. The succinimidyl group of these cross-linkers reacts with a primary amine, and the thiol-reactive maleimide forms a covalent bond with the thiol of a cysteine residue.

Cross-linking reagents often have low solubility in water. A hydrophilic moiety, such as a sulfonate group, may be added to the cross-linking reagent to improve its water solubility. Sulfo-MBS and sulfo-SMCC are examples of cross-linking reagents modified for water solubility.

Many cross-linking reagents yield a conjugate that is essentially non-cleavable under cellular conditions. However, some cross-linking reagents contain a covalent bond, such as a disulfide, that is cleavable under cellular conditions. For example, Traut's reagent, dithiobis (succinimidylpropionate) ("DSP"), and N-succinimidyl 3-(2-pyridyldithio) propionate ("SPDP") are well-known cleavable cross-linkers. The use of a cleavable cross-linking reagent permits the cargo moiety to separate from the transport polypeptide after delivery into the target cell. Direct disulfide linkage may also be useful.

Numerous cross-linking reagents, including the ones discussed above, are commercially available. Detailed instructions for their use are readily available from the commercial suppliers. A general reference on protein cross-linking and conjugate preparation is: Wong, CHEMISTRY OF PROTEIN CONJUGATION AND CROSS-LINKING, CRC Press(1991).

Chemical cross-linking may include the use of spacer arms. Spacer arms provide intramolecular flexibility or adjust intramolecular distances between conjugated moieties and thereby may help preserve biological activity. A spacer arm may be in the form of a polypeptide moiety that includes spacer amino acids, *e.g*. proline. Alternatively, a spacer arm may be part of the cross-linking reagent, such as in "long-chain SPDP" (Pierce Chem. Co., Rockford, IL, cat. No. 21651 H).

Alternatively, the chimeric peptide can be produced as a fusion peptide that includes the trafficking sequence and the JNK inhibitor sequence which can conveniently be expressed in known suitable host cells. Fusion peptides, as described herein, can be formed and used in ways analogous to or readily adaptable from standard recombinant DNA techniques, as describe above. (ELISA) and other immunologically-mediated techniques known within the art. In a specific embodiment, selection of antibodies that are specific to a particular domain of a JNK inhibitor peptide is facilitated by generation of hybridomas that bind to the fragment of a JNK inhibitor peptide possessing such a domain. Antibodies that are specific for a domain within a JNK inhibitor peptide, or derivative, fragments, analogs or homologs thereof, are also provided herein.

The anti-JNK inhibitor peptide antibodies may be used in methods known within the art relating to the localization and/or quantitation of a JNK. inhibitor peptide (*e.g*., for use in measuring levels of the peptide within appropriate physiological samples, for use in diagnostic methods, for use in imaging the peptide, and the like). In a given embodiment, antibodies for the JNK inhibitor peptides, or derivatives, fragments, analogs or homologs thereof that contain the antibody derived binding domain, are utilized as pharmacologically active compounds (herein after "Therapeutics").

### USE OF PEPTIDES AS DEFINED ABOVE FOR PREPARATION OF A MEDICAMENT FOR TREATING OR PREVENTING DISORDERS

### Hearing Loss

Included in the invention is the use of inventive peptides for preparing a medicament for preventing or treating hearing loss by administering to a subject a Therapeutic, *i.e.,* an inventive permeable bioactive peptide as defined above where the peptide prevents damage to the hair cell stereocilia, hair cell apoptosis or neuronal apoptosis. Preferably, the Therapeutic is the peptide including SEQ ID NO: 3, 4, 5, 6, and 22 or being SEQ ID NO: 11, 12, 13, 14, 15, 16, 23, 24, 25 or, 26.

Exposure to loud noise causes noise-induced hearing loss (NIHL) by damaging the organs of the Corti, Damage an NIHL depends upon both the level of the noise and the duration of the exposure. Hearing loss may be temporary (TTS) if a repair mechanism is able to restore the organ of the Corti, However it becomes permanent (PTS) when hair cells or neurons die. Structural correlates to noise trauma are oftwo types: (1) mild damage of synapses and or hair cell stereocilia which can be repaired and accounts for TTS and recovery and (2) severe damage inducing hair cell and neuronal apoptosis which can not be repaired and accounts for PTS.

The Therapeutic is to be administered to the subject before exposure to a noise trauma, antibiotic or chemotherapeutic agent. Alternatively, the Therapeutic is to be administered after the subject is exposed to a noise trauma, antibiotic or chemotherapeutic agent.

A noise trauma is a noise which is sufficient to cause damage to the corti. For example a noise trauma us at least 70 dB SPL, at least 90dB SPL or at least 100 dB SPL, at least 120 dB SPL or at least 130 dB SPL.

Antibiotics include for example penicillins such as penicillin G, penicillin V, ampicillin, amoxicillin, dicloxacillin, and oxacillin; cephalosporins such as cephalexin (Keflex), cefaclor (Ceclor), and cefixime (Suprax); aminoglycoside such as tobramycin, and streptomycin; macrolides, such as erythromycin, azithromycin (Zithromax) and clarithromycin; sulfonamides such as trimethoprim-sulfamethoxazole or tetracylines such as tetracycline, or doxycycline.

### PHARMACEUTICAL COMPOSITIONS

The Therapeutics may include, *e.g.*: (i) any one or more of the inventive JNK inhibitor peptides including any one of the amino acid sequences selected from SEQ ID NOs: 3-6, and 22, being any one of the amino acid sequences selected from SEQ ID NOs: 11-16 and 23-26, or a peptide being at least 95% homologous to these peptides, and derivative, fragments, analogs and homologs thereof; and (ii) nucleic acids encoding these JNK inhibitor peptides, and derivatives, fragments, analogs and homologs thereof.

The term "therapeutically effective" means that the amount of inhibitor peptide, for example, which is used, is of sufficient quantity to ameliorate the JNK associated disorder, i.e., hearing loss without being encompassed by the present invention, the term treatment includes administration of a reagent that modulates JNK kinase activity, as defined above. The term "modulate" includes the suppression of expression of JNK when it is over-expressed. It also includes suppression of phosphorylation of c-jun, ATF2 or NFAT4, for example, by using a peptide including the amine acid sequences of SEQ ID NOs: 3-6, and 22, or being SEQ ID NOs: 11-16 and 23-26 as a competitive inhibitor of the natural c-jun ATF2 and NFAT4 binding site in a cell. Thus also includes suppression of hetero- and homo-meric complexes of transcription factors made up of c-jun, ATF2, or NFAT4 and their related partners, such as for example the AP-1 complex that is made up of c-jun, AFT2 and c-fos.

In some instances, "modulate" may include the increase of JNK expression, for example by use of an IB peptide-specific antibody that blocks the binding of an IB-peptide to JNK, thus preventing JNK inhibition by the IB-related peptide. The JNK inhibitor, peptides, fusion peptides and nucleic acids of the invention can be formulated in pharmaceutical compositions. These compositions may comprise, in addition to one of the above substances, a pharmaceutically acceptable excipient, carrier, buffer, stabiliser or other materials well known to those skilled in the art. Such materials should be non-toxic and should not interfere with the efficacy of the active ingredient. The precise nature of the carrier or other material may depend on the route of administration, *e.g.* oral, intravenous, cutaneous or subcutaneous, nasal, intramuscular, intraperitoneal, intrauricular ,or patch routes.

Pharmaceutical compositions for oral administration may be in tablet, capsule, powder or liquid form. A tablet may include a solid carrier such as gelatin or an adjuvant. Liquid pharmaceutical compositions generally include a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringers Injection. Preservatives, stabilisers, buffers, antioxidants and/or other additives may be included, as required.

Whether it is a polypeptide, peptide, or nucleic acid molecule, according to the present invention that is to be given to an individual, administration is preferably in a "prophylactically effective amount" or a "therapeutically effective amount" (as the case may be, although prophylaxis may be considered therapy), this being sufficient to show benefit to the individual. The actual amount to be administered, and rate and time-course of administration, will depend on the nature and severity of what is being treated. Prescription of treatment, *e.g.* decisions on dosage etc, is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in REMINGTON'S PHARMACEUTICAL SCIENCES, 16th edition, Osol, A. (ed),1980.

Alternatively, targeting therapies may be used to deliver the active agent more specifically to certain types of cell, by the use of targeting systems such as antibody or cell specific ligands. Targeting may be desirable for a variety of reasons; for example if the agent is unacceptably toxic, or if it would otherwise require too high a dosage, or if it would not otherwise be able to enter the target cells.

Instead of administering these agents directly, they could be produced in the target cells by expression from an encoding gene introduced into the cells, *e.g.* in a viral vector (a variant of the VDEPT technique - *see* below). The vector could be targeted to the specific cells to be treated, or it could contain regulatory elements, which are switched on more or less selectively by the target cells.

Alternatively, the agent could be administered in a precursor form, for conversion to the active form by an activating agent produced in, or targeted to, the cells to be treated. This type of approach is sometimes known as ADEPT or VDEPT; the former involving targeting the activating agent to the cells by conjugation to a cell-specific antibody, while the latter involves producing the activating agent, *e.g.* a JNK inhibitor peptide, in a vector by expression from encoding DNA in a viral vector (*see* for example, EP-A-415731 and WO 90/07936)_{.}

In a specific embodiment of the present invention, nucleic acids including a sequence that encodes a JNK inhibitor peptide including any one of the amine acid sequences selected from SEQ ID NOs: 3-6 and 22, or being any one of the amino acid sequences selected from SEQ ID NOs: 11-16 and 23-26, on a peptide being at least about 95% homologous to these peptides, or functional derivatives thereof, may be administered with a pharmaceutical composition to modulate activated JNK signaling pathways by way of gene therapy. In more specific embodiments, a nucleic acid or nucleic acids encoding a JNK inhibitor peptide as defined above, , may be administered by way of gene therapy. Gene therapy refers to therapy that is performed by the administration of a specific nucleic acid to a subject. In this embodiment of the present invention, the nucleic acid produces its encoded peptide(s), which then serve(s) to exert a therapeutic effect by modulating function of the disease or disorder i.e. hearing loss. Any of the methodologies relating to gene therapy available within the art may be used therefore. *See e.g.,* Goldspiel, et al., 1993. Clin. Pharm 12: 488-505.

Therapeutics may further comprises a nucleic acid that is part of an expression vector expressing any one or more of the IB-related peptides, the JBD₂₀-related peptides or fragments, derivatives or analogs thereof as defined above, within a suitable host. Such a nucleic acid may possess a promoter that is operably-linked to coding region(s) of a JNK inhibitor peptide. The promoter may be inducible or constitutive, and, optionally, tissue-specific. The nucleic acid molecule may further comprise coding sequences (and any other desired sequences) which are flanked by regions that promote homologous recombination at a desired site within the genome, thus providing for intra-chromosomal expression of nucleic acids. *See e.g.,* Koller and Smithies, 1989. Proc Natl Acad Sci USA 86: 8932-8935.

Delivery of such a Therapeutic nucleic acid into a patient may be either direct (*i.e.*, the patient is directly exposed to the nucleic acid or nucleic acid-containing vector) or indirect (*i.e.*, cells are first transformed with the nucleic acid *in vitro,* then transplanted into the patient). These two approaches are known, respectively, as *in vivo* or *ex vivo* gene therapy. Such a nucleic acid may be directly administered *in vivo*, where it is expressed to produce the encoded product. This may be accomplished by any of numerous methods known in the art including, *e.g.*, constructing the nucleic acid as part of an appropriate nucleic acid expression vector and administering the same in a manner such that it becomes intracellular (e.g., by infection using a defective or attenuated retroviral or other viral vector; *see* U.S. Patent No. 4,980,286); directly injecting naked DNA; using microparticle bombardment (*e.g*., a "Gene Gun^{®}; Biolistic, DuPont); coating the nucleic acids with lipids; using associated cell-surface receptors/transfecting agents; encapsulating in liposomes, microparticles, or microcapsules; administering it in linkage to a peptide that is known to enter the nucleus; or by administering it in linkage to a ligand predisposed to receptor-mediated endocytosis (*see, e.g.,* Wu and Wu, 1987. J Biol Chem 262: 4429-4432), which can be used to "target" cell types that specifically express the receptors of interest, etc.

An additional illustrative approach to gene therapy may include transferring a gene into cells in *in vitro* tissue culture by such methods as electroporation, lipofection, calcium phosphate-mediated transfection, viral infection, or the like. Generally, the method of transfer includes the concomitant transfer of a selectable marker to the cells. The cells are then placed under selection pressure (*e.g.,* antibiotic resistance) so as to facilitate the isolation of those cells that have taken up, and are expressing, the transferred gene. Those cells may be delivered to a patient. In a specific embodiment, prior to the *in vivo* administration of the resulting recombinant cell, the nucleic acid is introduced into a cell by any method known within the art including, *e.g.,* transfection, electroporation, microinjection, infection with a viral or bacteriophage vector containing the nucleic acid sequences of interest, cell fusion, chromosome-mediated gene transfer, microcell-mediated gene transfer, spheroplast fusion, and similar methodologies that ensure that the necessary developmental and physiological functions of the recipient cells are not disrupted by the transfer. *See e.g.,* Loeffler and Behr, 1993. Meth Enzymol 217: 599-618. The chosen technique should provide for the stable transfer of the nucleic acid to the cell, such that the nucleic acid is expressible by the cell. Typically, the transferred nucleic acid is heritable and expressible by the cell progeny.

Those resulting recombinant cells may be delivered to a patient by various methods known within the art including, *e.g.*, injection of epithelial cells (e.g., subcutaneously), application of recombinant skin cells as a skin graft onto the patient, and intravenous injection of recombinant blood cells (e.g., hematopoietic stem or progenitor cells). The total amount of cells that are envisioned for use depend upon the desired effect, patient state, and the like, and may be determined by one skilled within the art.

Cells into which a nucleic acid can be introduced for the above illustrative purposes of gene therapy may encompass any desired, available cell type, and may be xenogeneic, heterogeneic, syngeneic, or autogeneic. Cell types may include differentiated cells such as epithelial cells, endothelial cells, keratinocytes, fibroblasts, muscle cells, hepatocytes and blood cells, or various stem or progenitor cells, in particular embryonic heart muscle cells, liver stem cells (International Patent Publication WO 94/08598), neural stem cells (Stemple and Anderson, 1992, Cell 71: 973-985), hematopoietic stem or progenitor cells, *e.g.*, as obtained from bone marrow, umbilical cord blood, peripheral blood, fetal liver, and the like. Cells utilized for gene therapy may be further autologous to the patient.

### IMMUNOASSAYS

The peptides of the present invention may be utilized in assays (*e.g.*, immunoassays) to detect, prognose, diagnose, or monitor the above conditions, diseases, and disorders characterized by aberrant levels of JNK, or a JNK inhibitor peptide, or monitor the treatment thereof. An "aberrant level" means an increased or decreased level in a sample relative to that present in an analogous sample from an unaffected part of the body, or from a subject not having the disorder. The immunoassay may be performed by a method comprising contacting a sample derived from a patient with an antibody under conditions such that immunospecific-binding may occur, and subsequently detecting or measuring the amount of any immunospecific-binding by the antibody. E.g an antibody specific for a JNK inhibitor peptide may be used to analyze a tissue or serum sample from a patient for the presence of JNK or a JNK inhibitor peptide; wherein an aberrant level of JNK or a JNK inhibitor peptide is indicative of a diseased condition. The immunoassays that may be utilized include competitive and non-competitive assay systems using techniques such as Western Blots, radioimmunoassays (RIA), enzyme linked immunosorbent assay (ELISA), "sandwich" immunoassays, immunoprecipitation assays, precipitin reactions, gel diffusion precipitin reactions, immunodiffusion assays, agglutination assays, fluorescent immunoassays, complement-fixation assays, immunoradiometric assays, and protein-A immunoassays, etc.

The present invention is not to be limited in scope by the specific embodiments described herein. Indeed, various modifications of the invention in addition to those described herein will become apparent to those skilled in the art from the foregoing description and accompanying figures.

### SPECIFIC EXAMPLES

### Example 1: Identification of JNK Inhibitor Peptides

Amino acid sequences important for efficient interaction with JNK were identified by sequence alignments between known JBDs. Sequence comparison between the JBDs of IB1 [SEQ ID NO:17], IB2 [SEQ ID NO:18], c-Jun [SEQ ID NO:19] and ATF2 [SEQ ID NO:20] defined a weakly conserved 8 amino acid sequence (FIG. 1A). Since the JBDs of IB1 and IB2 are approximately 100 fold as efficient as c-Jun or ATF2 in binding JNK (Dickens et al. Science 277: 693 (1997), it was reasoned that conserved residues between IB1 and IB2 must be important to confer maximal binding. The comparison between the JBDs of 1B1 and IB2 defined two blocks of seven and three amino acids that are highly conserved between the two sequences. These two blocks are contained within a peptide sequence of 23 amino acids in IB1 [SEQ ID NO:1] and 21 amino acid IB2 [SEQ ID NO:2]. These sequences are shown in FIG. 1B, dashes in the IB2 sequence indicate a gap in the sequence in order to align the conserved residues.

The JNK inhibitor (JNKI) peptides of the present invention were obtained by linking the 20 amino acid JNK-binding motif of JIP-1/IB1, referred to herein as JBD₂₀, to a trafficking protein, such as for example, the 10 amino acid HIV-TAT₄₈₋₅₇ transporter sequence.

### Example 2: Preparation of JNK Inhibitor Fusion Proteins

JNK inhibitor fusion proteins were synthesized by covalently linking the C-terminal end of JBD₂₃ or the 21 amino acid sequence derived from the JBD of IB2 (JBD₂₁) or the C-terminal end of the JBD₂₀ amino acid sequence to a N-terminal 10 amino acid long carrier peptide derived from the HIV-TAT₄₈₋₅₇(Vives et al., J. Biol. Chem. 272: 16010 (1997)) via a spacer consisting of two proline residues. This spacer was used to allow for maximal flexibility and prevent unwanted secondary structural changes. As shown in FIG. IC, these preparations were designated L-TAT [SEQ ID NO:7], L-TAT-IB1 [SEQ ID NO:11],L-TAT-IB2 [SEQ ID NO:12] and L-TAT-JNKI1 [SEQ ID NO:21], respectively. All-D retro-inverso peptides TAT-fusion peptides were also synthesized and were designated D-TAT [SEQ ID NO:8], D-TAT-IB1 [SEQ ID NO:14], AND D-TAT-JNKI1 [SEQ ID NO:22] respectively. All D and L peptides were produced by classical F-mock synthesis and further analysed by Mass Spectrometry. They were finally purified by HPLC. To determine the effects of the proline spacer, two types of TAT peptide were produced one with and one without two prolines. The addition of the two prolines did not appear to modify the entry or the localization of the TAT peptide inside cells.

Generic peptides showing the conserved amino acid residues are given in FIG. 2. An "X" indicates any amino acid. The number of Xs in a given peptide is not limited to the one depicted, and may vary (*i.e.*, X can represent any number of amino acid residues, including zero). See above for a more detailed description of the generic sequences.

### Example 3: Inhibition of βCell Death By JBD₂₃

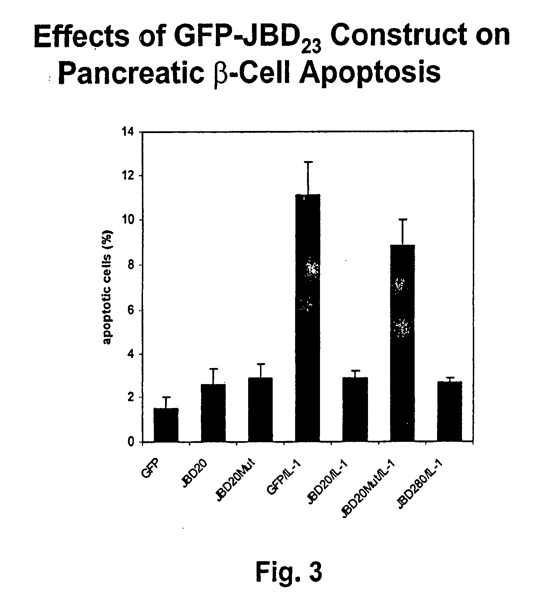

Effects of the 23 a.a. long JBD sequence of IB1 on JNK biological activities were then studied. The 23 a.a. sequence was linked N-terminal to the Green Fluorescent Protein (GFP-JBD₂₃ construct), and the effect of this construct on pancreatic β-cell apoptosis induced by IL-1β was evaluated. See FIG. 3. This mode of apoptosis was previously shown to be blocked by transfection with JBD₁₋₂₈₀, whereas specific inhibitors of ERK1/2 or p3 8 did not protect See Ammendrup *et al, supra.*

Oligonucleotides corresponding to the 23 amino acid sequence (JBD₂₃; FIG 1B) and a sequence mutated at the fully conserved regions (JBD₂₃ₘᵤₜ) were synthesized and directionally inserted into the EcoRI and SalI sites of the pEGFP-N1 vector encoding the Green Fluorescent Protein (GFP) (from Clontech). Insulin producing βTC-3 cells were cultured in RPMI 1640 medium supplemented with 10% Fetal Calf Serum, 100 µg/mL Streptomycin,100 units/mL Penicillin and 2 mM Glutamine. Insulin producing βTC-3 cells were transfected with the indicated vectors and IL-1 β (10 ng/mL) was added to the cell culture medium. The number of apoptotic cells were counted at 48 hours after the addition of IL-1β using an inverted fluorescence microscope. Apoptotic cells were discriminated from normal cells by the characteristic "blebbing out" of the cytoplasm were counted after two days.

As indicated in FIG. 3, GFP is Green Fluorescent protein expression vector used as a control; JBD23 is the vector expressing a chimeric GFP linked to the 23 a.a. sequence from the JBD of IB1; JBD23Mut is the same vector as GFP-JBD23, but with a JBD mutated at four conserved residues shown as FIG 1B; and JBD280 is the GFP vector linked to the entire JBD (a.a. 1-280). The GFP-JBD₂₃ expressing construct prevented IL-1β induced pancreatic β-cell apoptosis as efficiently as the entire JBD₁₋₂₈₀ (FIG. 3, JBD23/IL-1 compared to JBD280/IL-1). As additional controls, sequences mutated at fully conserved IB1 residues had greatly decreased ability to prevent apoptosis (FIG. 3, JBD23Mut/IL-1).

### Example 4: Cellular Import Of TAT-IB1 And TAT-IB2 Peptides

The ability of the L- and D-enantiomeric forms of TAT, TAT-IB1 and TAT-IB2 peptides ("TAT-IB peptides") to enter cells were evaluated.

L-TAT, D-TAT, L-TAT-IB1, L-TAT-IB2 and D-TAT-IB1 peptides [SEQ ID NOs:7, 8, 11, 12 and 14, respectively] were labeled by N-terminal addition of a glycine residue conjugated to fluorescein. Labeled peptides (1 µM) were added to βTC-3 cell cultures, which were maintained as described in Example 3. At predetermined times, cells were washed with PBS and fixed for five minutes in ice-cold metbanol-acetone (1:1) before being examined under a fluorescence microscope. Fluorescein-labeled BSA (1 µM, 12 moles/mole BSA) was used as a control. Results demonstrated that all the above fluorescein labeled peptides had efficiently and rapidly (less than five minutes) entered cells once added to the culture medium. Conversely, fluorescein labeled bovine serum albumin (1µM BSA, 12 moles fluorescein/mole BSA) did not enter the cells.

A time course study indicated that the intensity of the fluorescent signal for the L-enantiomeric peptides decreased by 70% following a 24 hours period. Little to no signal was present at 48 hours. In contrast, D-TAT and D-TAT-EB1 were extremely stable inside the cells. Fluorescent signals from these all-D retro-inverso peptides were still very strong 1 week later, and the signal was only slightly diminish at 2 weeks post treatment.

### Example 5: In Vitro Inhibition Of c-JUN, ATF2 and Elk1 Phosphorylation

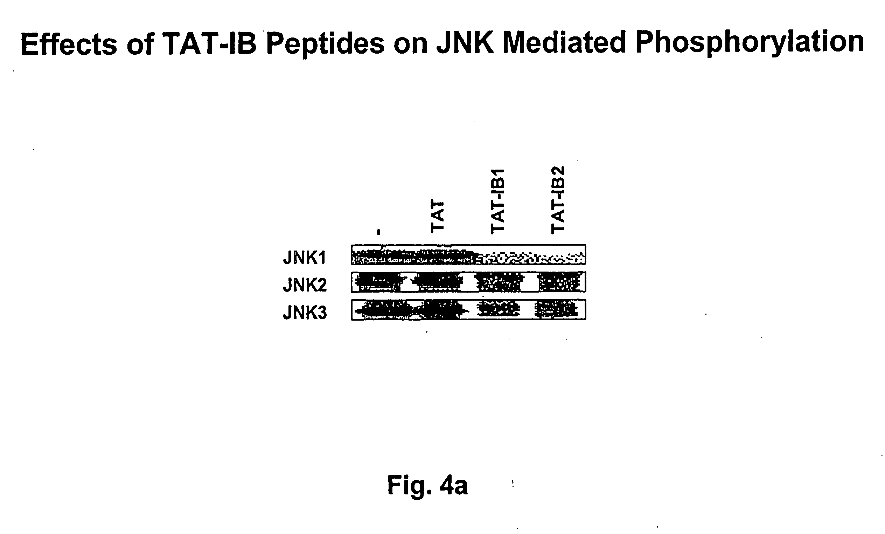

The effects of the peptides on JNKs-mediate phosphorylation of their target transcription factors were investigated *in vitro.* Recombinant and nonactivated JNK1, JNK2 and JNK3 were produced using a TRANSCRIPTION AND TRANSLATION rabbit reticulocyte lysate kit (Promega) and used in solid phase kinase assays with c-Jun, ATF2 and Elk1, either alone or fused to glutathione-S-transferase (GST), as substrates. Dose response studies were performed wherein L-TAT, L-TAT-IB1 or L-TAT-IB2 peptides (0-25 µM) were mixed with the recombinant JNK1, JNK2, or JNK3 kinases in reaction buffer (20 mM Tris-acetate,1mM EGTA, 10mM p-nitrophenyl-phosphate (pNPP), 5 mM sodium pyrophosphate, 10 mM p-glycerophosphate, 1mM dithiothreitol) for 20 minutes. The kinase reactions were then initiated by the addition of 10 mM MgCl₂ and 5 µCi ³³P-γ-dATP and 1 µg of either GST-Jun (a.a. 1-89), GST-AFT2 (a.a. 1-96) or GST-ELK1 (a.a. 307-428). GST-fusion proteins were purchased from Stratagene (La Jolla, CA). Ten µL of glutathione-agarose beads were also added to the mixture. Reaction products were then separated by SDS-PAGE on a denaturing 10 % polyacrylamide gel. Gels were dried and subsequently exposed to X-ray films (Kodak). Nearly complete inhibition of c-Jun, ATF2 and Elk1 phosphorylation by JNKs was observed at TAT-IB peptide doses as low as 2.5 µM. However, a marked exception was the absence of TAT-IB inhibition of JNK3 phosphorylation of Elk1. Overall, the TAT-IB1 peptide appeared slightly superior to TAT-IB2 in inhibiting JNK family phosphorylation of their target transcription factors. (*See,* FIG. 4A).

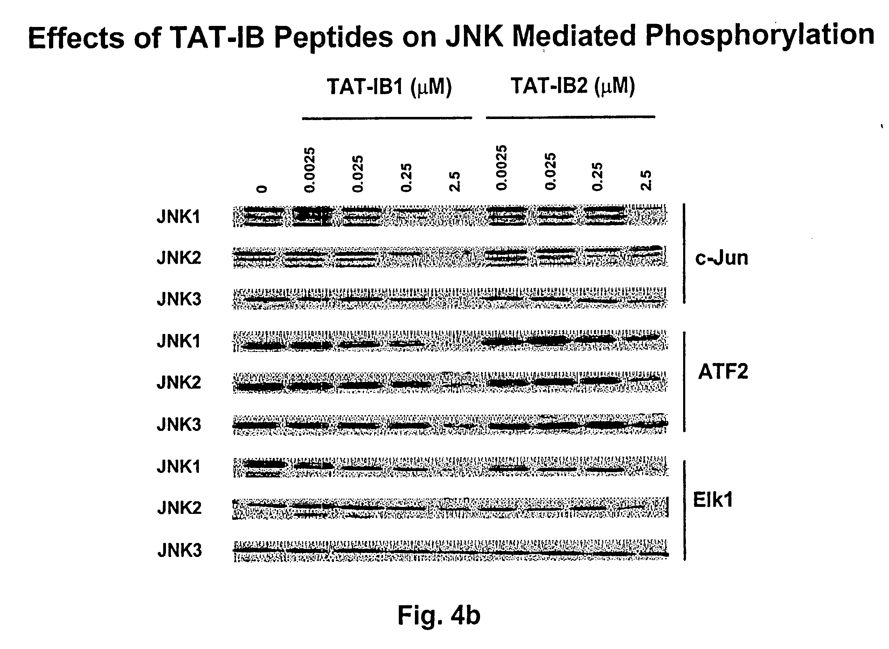

The ability of D-TAT, D-TAT-IB1 and L-TAT-IB1 peptides (0-250 µM dosage study) to inhibit GST-Jun (a.a. 1-73) phosphorylation by recombinant JNK1, JNK2, and JNK3 by were analyzed as described above. Overall, D-TAT-IB1 peptide decreased JNK-mediated phosphorylation of c-Jun, but at levels approximately 10-20 fold less efficiently than L-TAT-IB1. (*See,* FIG. 4B).

### Example 6: Inhibition of c-JUN Phosphorylation By Activated JNKs

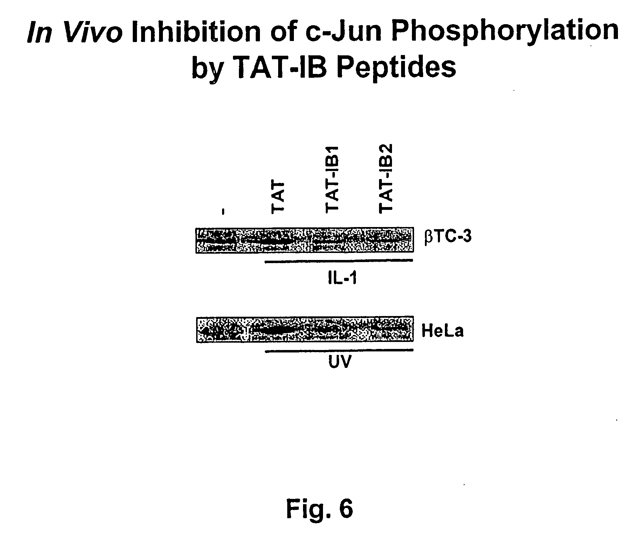

The effects of the L-TAT, L-TAT-IB1 or L-TAT-IB2 peptides on JNKs activated by stressful stimuli were evaluated using GST-Jun to pull down JNKs from UV-Jight irradiated HeLa cells or IL-1β treated βTC cells. βTC cells were cultured as described above. HeLa cells were cultured in DMEM medium supplemented with 10 % Fetal Calf Serum, 100 µg/mL Streptomycin, 100 units/ml Penicillin and 2 mM Glutamine. One hour prior to being used for cell extract preparation, βTC cells were activated with IL-1β as described above, whereas HeLa cells were activated by UV-light (20 J/m²). Cell extracts were prepared from control, UV-light irradiated HeLa cells and IL-1β treated βTC-3 cells by scraping the cell cultures in lysis buffer (20 mM Tris-acetate,1mM EGTA, 1% Triton X-100, 10 mM p-nitrophenyl-phosphate, 5 mM sodium pyrophosphate, 10 mM β-glycerophosphate, 1 mM dithiothretiol). Debris was removed by centrifugation for five minutes at 15,000 rpm in an SS-34 Beckman rotor. One-hundred µg extracts were incubated for one hour at room temperature with one µg GST-jun (amino acids 1-89) and 10 µL of glutathione-agarose beads (Sigma). Following four washes with the scraping buffer, the beads were resuspended in the same buffer supplemented with L-TAT, L-TAT-IB1 or L-TAT-IB2 peptides (25 µM) for 20 minutes. Kinase reactions were then initiated by the addition of 10 mM MgCl₂ and 5 µCi ³³P-γ-dATP and incubated for 30 minutes at 30°C. Reaction products were then separated by SDS-PAGE on a denaturing 10 % polyacrylamide gel. Gels were dried and subsequently exposed to X-ray films (Kodak). The TAT-IB peptides efficiently prevented phosphorylation of c-Jun by activated JNKs in these experiments.. (*See*, FIG. 6).

### Example 7: In vivo inhibition of c-JUN Phosphorylation By TAT-IB Peptides

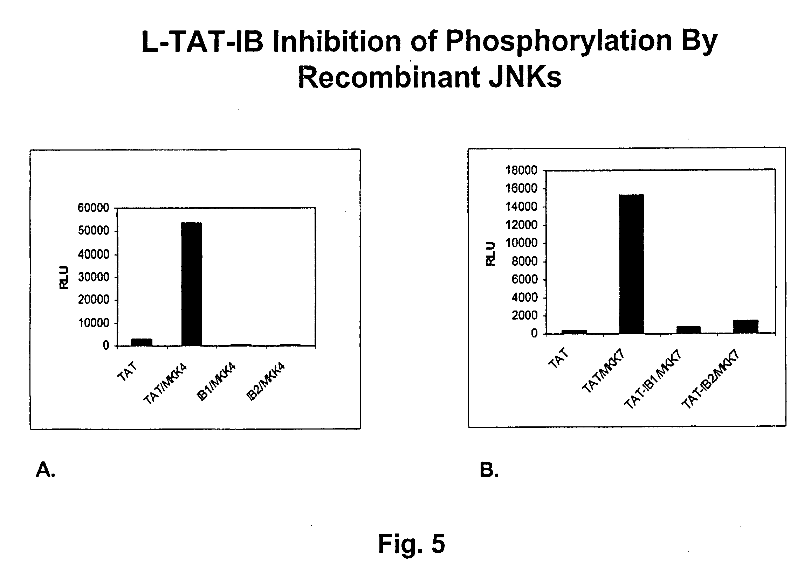

To determine whether the cell-permeable peptides could block JNK signaling *in vivo*, we used a heterologous GAL4 system. HeLa cells, cultured as described above, were co-transfected with the 5xGAL-LUC reporter vector together with the GAL-Jun expression construct (Stratagene) comprising the activation domain of c-Jun (amino acids 1-89) linked to the GAL4 DNA-binding domain. Activation of JNK was achieved by the co-transfection of vectors expressing the directly upstream kinases MKK4 and MKK7 (*See*, Whitmarsh *et al.,* Science 285: 1573 (1999)). Briefly, 3x10⁵ cells were transfected with the plasmids in 3.5-cm dishes using DOTAP (Boehringer Mannheim) following instructions from the manufacture. For experiments involving GAL-Jun, 20 ng of the plasmid was transfected with 1 µg of the reporter plasmid pFR-Luc (Stratagene) and 0.5 µg of either MKK4 or MKK7 expressing plasmids. Three hours following transfection, cell media were changed and TAT, TAT-IB1, and TAT-IB2 peptides (1 µM) were added. The luciferase activities were measured 16 hours later using the "Dual Reporter System" from Promega after normalization to protein content. As shown in FIG. 5, addition of both the TAT-IB1 and TAT-IB2 peptides blocked activation of c-Jun following MKK4 and MKK7 mediated activation of JNK. Because HeLa cells express both JNK1 and JNK2 isoforms but not JNK3, we transfected cells with JNK3. Again, the two TAT-IB peptides inhibited JNK2 mediated activation of c-Jun.

### Example 8: Inhibition Of IL-1β Induced Pancreatic β-Cell Death By TAT-IB Peptides

We investigated the effects of the L-TAT-IB peptides on the promotion of pancreatic β-cell apoptosis elicited by IL-1β. βTC-3 cell cultures were incubated for 30 minutes with 1 µM of either L-TAT-IB1 or L-TAT-IB2 peptides followed by 10 ng/mL of IL-1β. A second addition of peptide (1 µM) was performed 24 hours later. Apoptotic cells were counted after two days of incubation with IL-1β using Propidium Iodide (red stained cell are dead cells) and Hoechst 33342 (blue stained cell are cells with intact plasma membrane) nuclear staining. As shown in FIG. 5, addition of the TAT-IB peptides inhibited IL-1β-induced apoptosis of βTC-3 cells cultured in the presence of IL-1β for two

Long term inhibition of IL-1β induced cells death was examined by treating βTC-3 cells as described above, except that incubation of the cells with the peptides and IL-1β was sustained for 12 days. Additional peptides (1 µM) were added each day and additional IL-1β (10 ng/mL) was added every 2 days. The TAT IB1 peptide confers strong protection against apoptosis in these conditions. Taken together, these experiments establish that TAT-IB peptides are biologically active molecules able to prevent the effects of JNK signaling on cell fate.

### Example 9: Synthesis of an All-D-retro-inverso Peptides

Peptides of the invention may be all-D amino acid peptides synthesized in reverse to prevent natural proteolysis (*i.e.*, all-D-retro-inverso peptides). An all-D retro-inverso peptide of the invention would provide a peptide with functional properties similar to the native peptide, wherein the side groups of the component amino acids would correspond to the native peptide alignment, but would retain a protease resistant backbone.

Retro-inverso peptides of the invention are analogs synthesized using D-amino acids by attaching the amino acids in a peptide chain such that the sequence of amino acids in the retro-inverso peptide analog is exactly opposite of that in the selected peptide which serves as the model. To illustrate, if the naturally occurring TAT protein (formed of L-amino acids) has the sequence GRKKRRQRRR [SEQ ID NO:7], the retro-inverso peptide analog of this peptide (formed of D-amino acids) would have the sequence RRRQRRKKRG [SEQ ID NO:8]. The procedures for synthesizing a chain of D-amimo acids to form the retro-inverso peptides are known in the art. *See*, *e.g.,* Jameson et al., Nature, 368, 744-746 (1994); Brady et al., Nature, 368, 692-693 (1994)); Guichard et al., J. Med. Chem. 39, 2030-2039 (1996). Specifically, the retro- peptides were produced by classical F-mock synthesis and further analysed by Mass Spectrometry. They were finally purified by HPLC.

Since an inherent problem with native peptides is degradation by natural proteases and inherent immunogenicity, the heterobivalent or heteromultivalent compounds of this invention will be prepared to include the "retro-inverso isomer" of the desired peptide. Protecting the peptide from natural proteolysis should therefore increase the effectiveness of the specific heterobivalent or heteromultivalent compound, both by prolonging half-life and decreasing the extent of the immune response aimed at actively destroying the peptides.

### Example 10: Long term biological activity of all -D-retro-inverso IB Peptides

Long term biological activity is predicted for the D-TAT-IB retro-inverso containing peptide heteroconjugate when compared to the native L-amino acid analog owing to protection of the D-TAT-IB peptide from degradation by native proteases, as shown in Example 5.

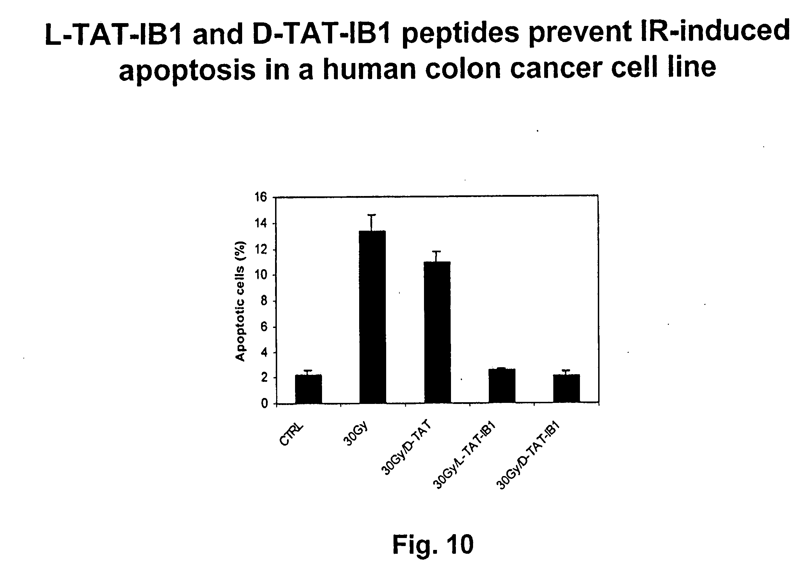

Inhibition of IL-1β induced pancreatic β-cell death by the D-TAT-IB1 peptide was analyzed. As shown in FIG. 10, βTC-3 cells were incubated as described above for 30 minutes with one single addition of the indicated peptides (1µM), then IL-1β (10 ng/ml) was added. Apoptotic cells were then counted after two days of incubation with IL-1β by use of Propidium Iodide and Hoechst 33342 nuclear staining. A minimum of 1,000 cells were counted for each experiment. Standard Error of the Means (SEM) are indicated, n=5. The D-TAT-IB1 peptide decreased IL-1 induced apoptosis to a similar extent as L-TAT-IB peptides (compare FIG. 5 and FIG. 10).

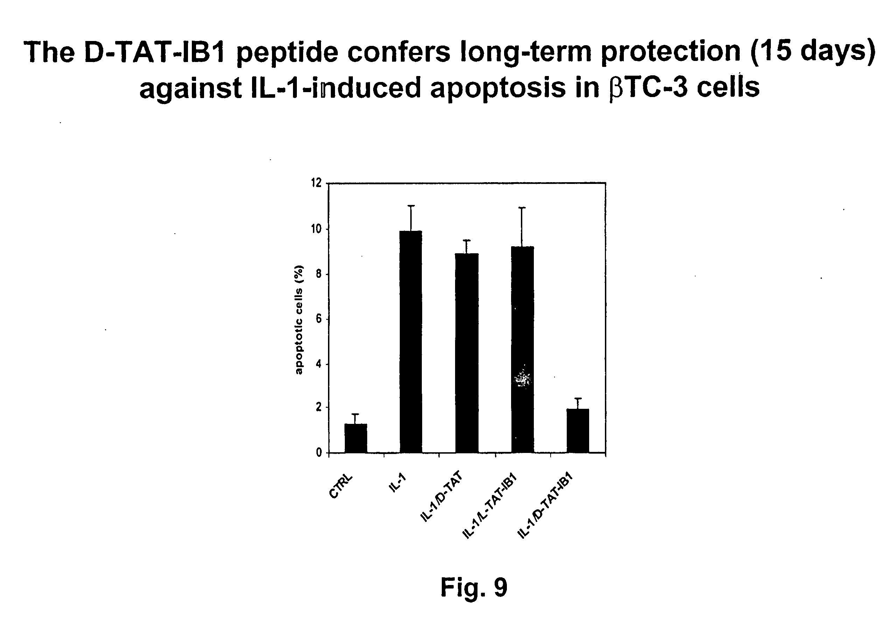

Long term inhibition of IL-1β induced cell-death by the D-TAT-IB1 peptide was also analyzed. βTC-3 cells were incubated as above for 30 minutes with one single addition of the indicated peptides (1µM), then IL-1β (10 ng/ml) was added, followed by addition of the cytokine every two days. Apoptotic cells were then counted after 15 days of incubation with IL-1β by use of Propidium Iodide and Hoechst 33342 nuclear staining. Note that one single addition of the L-TAT-IB1 peptide does not confer long-term protection. A minimum of 1,000 cells were counted for each experiment. Standard Error of the Means (SEM) are indicated, n=5. Results are shown in FIG.9. D-TAT-IB1, but not L-TAT-IB1, was able to confer long term (15 day) protection.

### Example 11: Inhibition Of Irradiation Induced Pancreatic β-Cell Death By TAT-IB Peptides

JNK is also activated by ionizing radiation. To determine whether TAT-IB peptides would provide protection against radiation-induced JNK damage, "WiDr" cells were irradiated (30Gy) in presence or absence of D-TAT, L-TAT-IB1 or D-TAT-IB1 peptides (1µM added 30 minutes before irradiation), as indicated in FIG. 10. Control cells (CTRL) were not irradiated. Cells were analyzed 48 hours later by mean of PI and Hoechst 33342 staining, as described above. n=3, SEM are indicated. L-TAT-IB1 and D-TAT-IB1 peptides were both able to prevent irradiation induced apoptosis in this human colon cancer cell line.

### Example 12: Radioprotection to Ionizing Radiation By TAT-IB Peptides

To determine the radioprotective effects of the TAT-IB peptides, C57 B1/6 mice (2 to 3 months old) were irradiated with a Phillips RT 250 R-ray at a dose rate of 0.74 Gy/min (17 mA, 0.5 mm Cu filter). Thirty minutes prior to irradiation, the animals were injected i.p. with either the TAT, L-TAT-IB1 and D-TAT-IB1 peptides (30 µl of a 1mM solution). Briefly, mice were irradiated as follows: mice were placed in small plastic boxes with the head lying outside the box. The animals were placed on their back under the irradiator, and their neck fixed in a small plastic tunnel to maintain their head in a correct position. The body was protected with lead. Prior to irradiation mice were maintained on standard pellet mouse chow, however post irradiation mice were fed with a semi-liquid food that was renewed each day.

The reaction of the lip mucosa was then scored by 2 independent observers according to the scoring system developed by Parkins *et al.* (parkins et al, Radiotherapy & Oncology, 1: 165-173, 1983), in which the erythema status as well as the presence of edema, desquamation and exudation was quoted. Additionally, animals were weighed before each recording of their erythema/edema status.

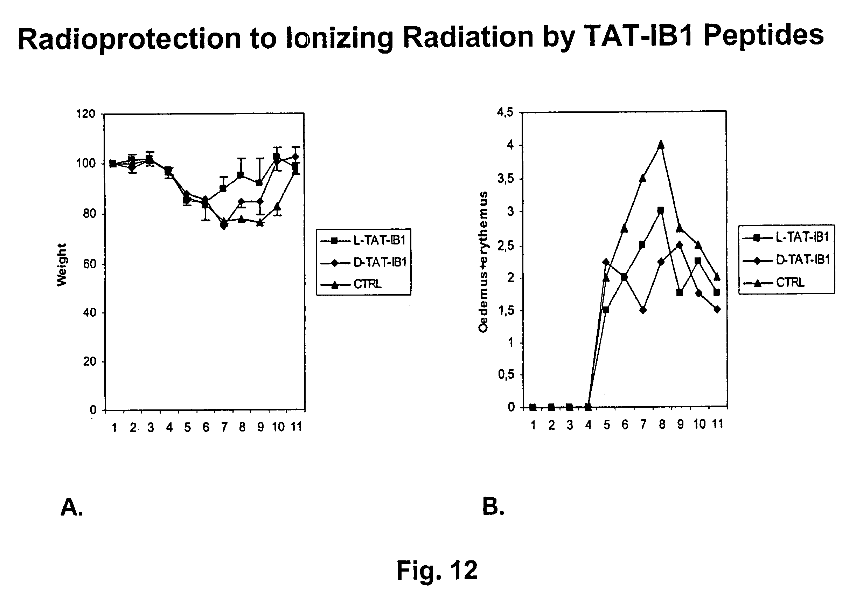

FIG.12A: illustrated the weight of the mice following irradiation. Values are reported to the initial weight of the mice that was set to 100. CTRL: control mice injected with 30 µl of a saline solution. n=2 for each values reported, S.D. are indicated. x values are days

FIG. 12B is illustrative of the erythema/edema scoring following irradiation. The edema and erythema status of the ventral lip of the same mice as in FIG. 12A was quantified. n=2 for each value reported. x values are days

The results of these experiments indicate that the TAT-IB Peptides can protect against weight loss and erythema/edema associated with ionizing radiation.

### Example 13: Suppression of JNK Transcription Factors by L-TAT-IB1 peptides

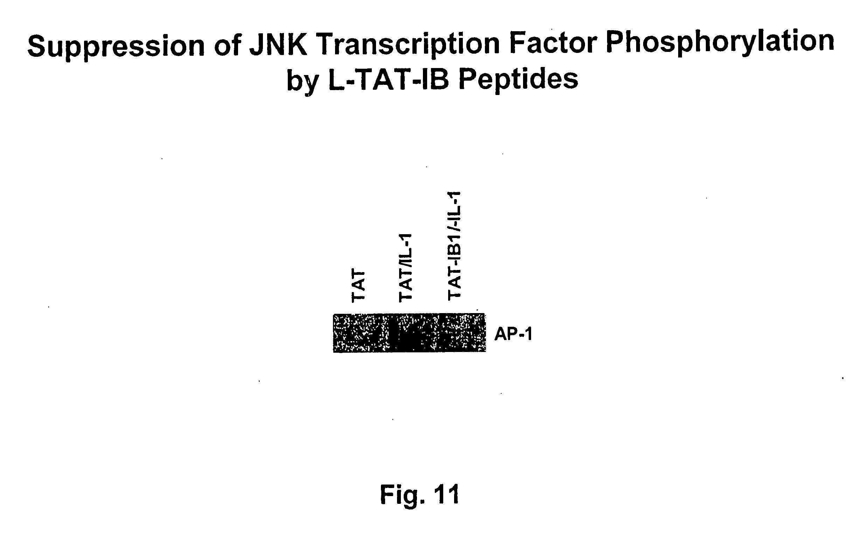

Gel retardation assays were carried out with an AP-1 doubled labeled probe ( 5'-CGC TTG ATG AGT CAG CCG GAA-3'. HeLa cell nuclear extracts that were treated or not for one hour with 5 ng/ml TNF-α, as indicated. TAT and L-TAT-IB1 peptides were added 30 minutes before TNF-α. Only the part of the gel with the specific AP-1 DNA complex (as demonstrated by competition experiments with non-labeled specific and non-specific competitors) is shown. L-TAT -IB1 peptides decrease the formation of the AP-1 DNA binding complex in the presence of TNF-α. (*See,* FIG 11).

### Example 14: Protection against noise-induced hearing loss by D-TAT-IB

### peptides

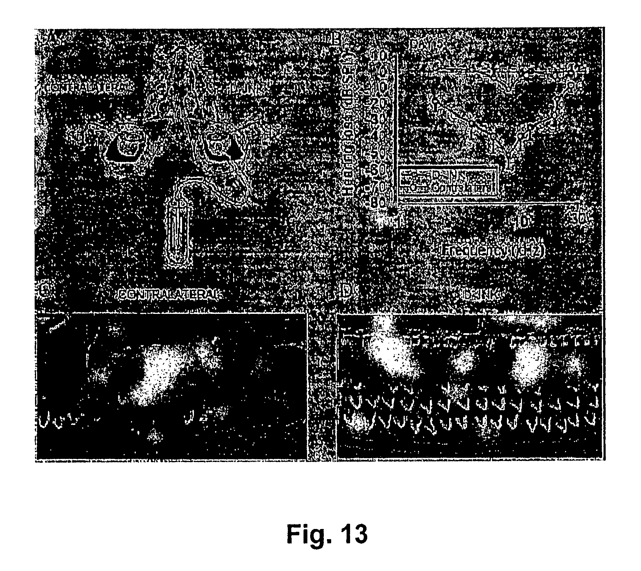

A solution of D-JNKI (1uM, 1ul/hr) was injected into the right internal ear of a guinea pig as shown in FIG 13, panel A, whereas the left ear was injected with saline only. The pig was then exposed to a noise trauma (120 db, 30 minutes), and recording of hearing sensitivity was performed three days after (FIG 13, panel B) as well as histological examination of the inner ear (FIG 13, panel C an D). As shown in FIG 13 the ciliated structures on the JNKI treated ear are completely protected from noise induced destruction as judged from the histological examination, in contrast to the non-treated ear where most of the ciliated structures have disappear. Furthermore, the sensitivity of the D-JNK1 treated ear to noise appear to be preserved (FIG 13, panel B).

### Example 15: Protection against antibiotic-induced hearing loss by D-TAT-IB peptides

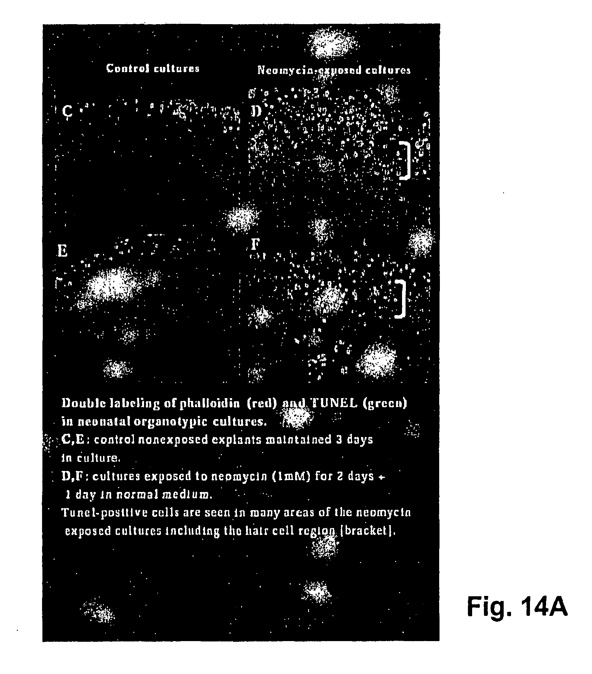

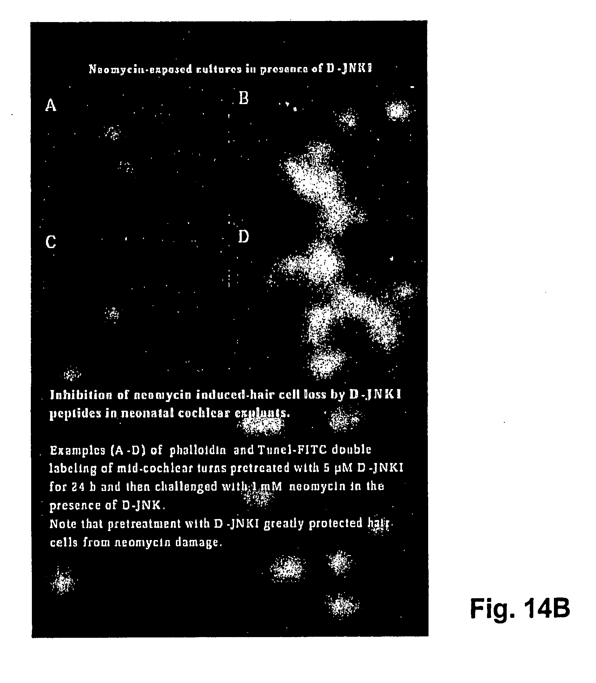

Chicken internal ears were treated with streptomycin in the presence/absence of D_JNKI. TUNEL experiments were then performed to detect apoptosis (green nuclei). As shown in FIG 14, D-JNKI fully protects internal ears from streptomycin induced apoptosis. Thus D-JNK-I is useful in the prevention of hearing loss conditions sustained by antibiotic therapy.

### Example 16: Protection against pancreatic islet destruction induced by proinflammatory cytokines by D-TAT-IB peptides

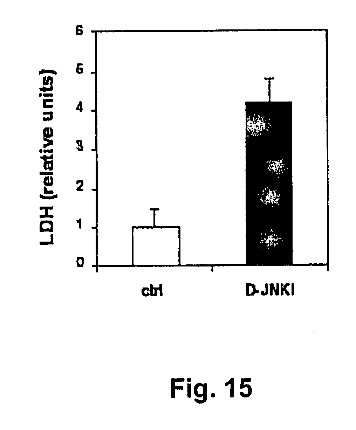

Pancreatic islets cells were treated with D-JNK1 (1mM for one hour before being exposed to interleukin 1B ( 10 ng/ml). As shown in FIG 15, D-JNKI treated islets resist IL-1B induced destruction. This indicates that treatment with D-JNKI helps preserve grafted islets.

### Example 17: Increase recovery of pancreatic islets cells by D-TAT-IB peptides

D-JNK-I were added together with collagenase during islet cell isolation. This resulted in an increased yield of islet after 3 days in culture as measured by the increase in lactate dehydrogenase. See FIG 15.

### Example 18: General methods used in testing the effects of JNKI peptides on JNK activation and JNK-related action

General Neuronal Culture: Small pieces of cortex from the brains of two day old rat pups were dissected and incubated with 200 units of papain for 30 minutes at 34 °C. Then, the neurons were plated at densities of approximately 1 x 10⁶ cells/plate on dishes that had been pro-coated with 100 µg/mL poly-D-lysine. The cells were cultured using a B27/Neurobasal (Life Technologies) culture medium, supplemented with 0.5m glutamine, 100 U/mL penicillin and 100µg/mL streptomycin.

Lactate dehydrogenase (LDH) cytotoxicity assay: The amount of LDH released into the culture medium was measured using the Cytotox 96 non-radioactive cytotoxicity assay kit (Promega).

GST-c-Jun pull-down and kinase assay: Cellular extracts were prepared by scraping cells in lysis buffer (20 mM Tris-acetate,1 mM EGTA, 1 % Triton X-100,10 mM p-nitrophenyl-phosphate, 5 mM sodium pyrophosphate,10 mM β-glycerophosphate, 1 mM dithiothreitol). 25 µg samples were incubated for 1 hour at room temperature with 1 µg GST-c-Jun (amino acid residues 1-89) and 10 µL glutathione-agarose beads (Sigma). The beads were washed four times and then resuspended in the lysis buffer described above. *In vitro* kinase assays were then performed using recombinant JNK1α1 and 0.5 µg of a substrate selected from the group consisting of GST-fusion proteins (*e.g.*, GST-Jun and GST-E1k1 fusion proteins), casein and histone (Sigma). Reactions were initiated with 10 mM MgCl₂ and 10 µM ATP in the presence of 5 µCi ³³P-ATP, and were incubated for 30 minutes at 30 degrees Celsius. The reaction products were separated by SDS-PAGE, and the gels were dried and then exposed to X-ray films (Kodak).

Western blots: Total protein extracts were obtained by scraping cells in lysis buffer (described above), separating the proteins on a 12% SDS polyacrylamide gel. The separated proteins were then transferred onto polyvinylidene fluoride (PVDF) membrane. Antibodies used in the Western blots described herein were obtained from Alexis.

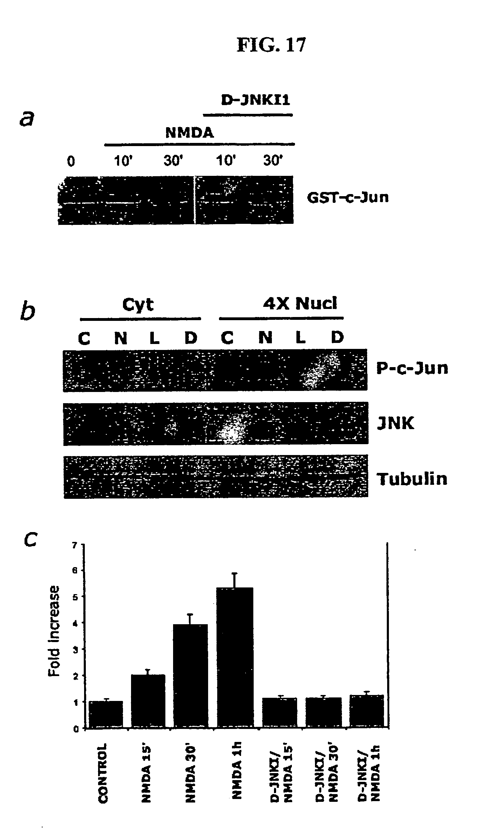

Separation of nuclei from cytoplasm: To isolate nuclei for Western blot analysis (see FIG. 17B), neurons were lysed for 15 minutes in lysis buffer, and then the samples were centrifuged at 300g for 10 minutes at 4°C. The nuclear pellets were reconstituted in lysis buffer and then sonicated.

Real-time RT-PCR: Real-time RT-PCR was performed using specific primers on a lightcycler apparatus (Roche). The housekeeping actin transcript was used to normalize for the amount and quality of the RNAs that were extracted by the Chomczynski method. *See* Chomczynski et al., Anal. Biochem., 162:156-59 (1987). The sequences of the primers used were as follows:

| | | |
|---|---|---|
| **c-Fos:** | Forward: | 5'-GCTGACAGATACACTCCAAG-3' |
| | Reverse: | 5'-CCTAGATGATGCCGGAAACA-3' |
| | | |
| **Actin:** | Forward: | 5'-AACGGCTCCGGCATGTGCAA-3' |
| | Reverse: | 5'-ATTGTAGAAGGTGTGGTGCCA-5' |

P-c-jun immunohistochemistry: P-c-jun, as used herein refers to phosphorylated forms of c-jun. P-c-jun was targeted with a rabbit polyclonal antibody (500x in PBS) (Cell Signaling Technology). The resulting antibody complex was visualized with 3,3-diaminobenzidine as the substrate.

Transient ischemia in adult mice: Using male ICR-CD1 mice (approximately 6 weeks old and weighing in the range of about 18 to 37 g) (Harlan, Inc.), ischemia was provoked by introducing a filament from the common carotid artery into the internal carotid artery and advancing the filament into the arterial circle, thereby occluding the middle cerebral artery. *See e.g.,* Huang et al, Science, 265:1883-85 (1994); Hara et al., Proc. Natl. Acad. Sci. (USA) 94:2007-12 (1997). Regional cerebral blood flow was measured by laser-Doppler flowmetry with a probe fixed on the skull throughout the ischemia and until 10 minutes after reperfusion. Rectal temperature was measured and maintained at 37°C. The animals were sacrificed 48 hours after reperfusion. Serial cryostat sections 20 µM-thick were traced using a computer-microscope system equipped with the Neurolucida program (Microbrightfield, Inc.), and the volumes of the ischemic area and of the whole brain were calculated (blind) with the Neuroexplorer program. Systolic and diastolic blood pressure were measured with an arterial catheter in three additional mice from 10 minutes before the D-JNKI1 injection until 30 minutes afterwards. These blood pressure measurements showed that the injections did not affect blood pressure (*i.e*., less than 10 % change). The Guidelines of the Swiss Federal Veterinary Office were followed in all experiments.

Permanent focal ischemia in young (P14) rats: Middle cerebral artery occlusion was obtained by electrocoagulating the middle cerebral artery at a position closed to its origin at the junction with the olfactory branch. The rats (from Wistar), which weighed in the range of about 27-35g, were sacrificed 24 hours after middle cerebral artery occlusion. The rats were sacrificed using an overdose of chloral hydrate and were perfused through the left ventricle with Zamboni's fixative. The brains were postfixed for 2 hours in the same solution used for perfusion, and then the brains were infiltrated overnight in 30% sucrose for cryoprotection. The outlines of each ischemic area were drawn on (stained) with a computer-microscope system. The area of the ischemic lesion and of the whole brain were traced from 50 µm serial cryostat sections stained with cresyl violet using the Neurolucida program, and the volumes of each were calculated using the Neuroexplorer program, as described above.

Statistics: Data from both ischemia models (*i.e.*, transient and permanent) were transformed logarithmically to satisfy the Gaussian criterion. Data was analyzed with an overall ANOVA (p<0.0001 for both models) followed by one-tailed unpaired t-tests.

### Example 19: Sensitivity and specificity of JNKI peptides against JNK action

The JNKI1 peptides used in these experiments are aimed at blocking the access of JNK to c-Jun and other substrates by a direct competitive mechanism. *See e.g.,* Bonny et al., Diabetes, 50:77-82 (2001); Barr et al., J. Biol. Chem., 277:10987-97 (2002).

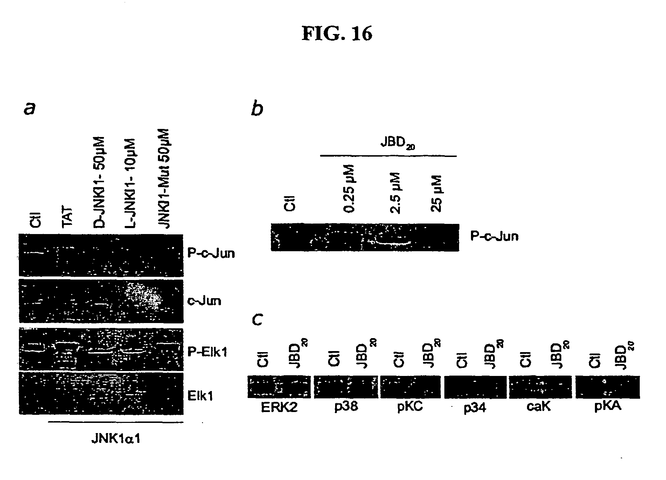

The inhibitory effect of L-JNKI1 and D-JNKI1 on JNK activation and action was tested using the kinase assays, as described above in Example 18. The results of these experiments are shown in FIGS. 16A-16C. The inhibitory effect of L-JNKI1 and D-JNKI1 on JNK activation and action is shown by their ability to prevent the phosphorylation *in vitro* of known JNK targets c-Jun and Elk1 using JNK1α1. (See FIG. 16A). The terms "P-Jun" and "P-Elk1," as used herein, refer to the radiolabeled (*i.e*., phosphorylated with ³³P-ATP) forms of GST-Jun and GST-Elk1 substrates, respectively. FIG. 16B demonstrates the inhibitory effect of the 20 amino acid minimal JNK-inhibitory sequence of JIP-IB1 (L-form of JBD₂₀ (SEQ ID NO:21)) in dose response experiments, using conditions similar to those used to test the inhibitory effect of L-JNKI1 and D-JNKI1 and using decreasing amounts of L-JBD₂₀. FIG. 16B illustrates that the L-JBD₂₀ peptide (SEQ ID NO:21) alone (*i.e,* without the TAT sequence) can inhibit JNK action. JBD₂₀ was also shown to inhibit other JNK targets including ATF2, IRS-1, MADD, bc1-x1. In each of these cases, the IC₅₀ was about 1 µM (data not shown). The TAT sequence was not linked to JBD₂₀ in these experiments, because, at concentration greater than 50 µM, the TAT sequence induces a nonspecific precipitation of the proteins in the extracts. Below 50 µM, TAT does not influence the inhibitory properties of the JBD₂₀ peptides.

*In vitro* experiments were performed to determine the specificity of the JNKI peptides in blocking JNK activation. In particular, the effect of these peptides on the activity of 40 different kinases (10 µM peptides, 10 µM ATP) towards their respective substrates was tested. The complete list of substrates used in these experiments can be found at http://www.upstate.com/img/pdf/KinaseProfiler.pdf. As expected, the JNKI peptides had an affect on the JNKs and MKK4 and MKK7 kinases, all of which contain JNK-binding domains. The peptides (both the L-JNKI1 and D-JNKI1 forms) completely failed to interfere with the activities of all other kinase. Additional experiments showed that 500 µM of the JBD₂₀ peptides did not interfere with the activity of 6 particular kinases: ERK2, p38, pKC, p34, caK and pKA (FIG.16C). The substrates for these kinases are ERK2:ERK1; p38:ATF2; p34, pKC, pKA:histone; and caK:caseine. This level of specificity is far above those achieved with other small chemical inhibitors of Jun-N-terminal kinase, thereby demonstrating the extremely high selectivity of the JNKI peptides of the invention. For a discussion of other small chemical inhibitors of the Jun N-terminal kinase (JNK), see Bennett et al., Proc. Natl. Acad Sci. (USA), 98:13681-86 (2001).

### Example 20: Effects of the JNKI peptides on JNK targets inside NMDA-treated cortical neurons

A series of experiments were performed to analyze the effects of the JNKI peptides of the invention on different JNK targets inside neurons. The activation of JNK in N-methyl-D-aspartate (NMDA)-treated cortical neurons in culture was estimated by performing kinase assays on pulled-down JNK using GST-c-Jun, using the methods described above. (*See e.g.*, Ko et al., J. Neurochem. 71:1390-1395 (1998); Coffey et al., J. Neurosci. 20:7602-7613 (2000) ). The results of these experiments are shown in FIGS.17-18.

FIG.17A shows the JNK activity in untreated neurons ("0"), after 10 minutes exposure to 100 µM NMDA (10') or after 30 minutes exposure to 100 µM NMDA. The two lanes at the right of FIG.17C demonstrate that JNK activation was essentially unchanged by D-JNKI1. The increase in JNK activity appeared maximal (*i.e*., 2.2 fold) after 30 minutes of NMDA treatment (FIG. 17A). This increase in JNK activity translated into an elevated c-Jun phosphorylation (FIG. 17B). Addition of the cell-penetrating peptides L-JNKI1 and D-JNKI1 was shown to completely prevent the increase in P-c-Jun after 5 hours of exposure to 100 µM NMDA, despite a normal level of JNK activation. Addition of L-JNKI1 and D-JNKI1 brought the level of P-c-Jun below even the level of P-c-Jun in the control.

NMDA-induced transcription of the c-fos gene, under the influence of JNK via the Elkl transcription factor was also completely prevented by the addition of L-JNKI and D-JNKI1 (FIG. 17C). c-fos expression was quantitated by real-time PCR (Lightcycler) using RNA extracted using the methods described above in Example 18. The data in FIG.17C is presented as c-fos expression relative to actin (n=4). For a description of the induction of c-fos expression through JNK-mediated TCF/Elk-1 phosphorylation, see Cavigelli et al., EMBOJ.,14:5957-5964 (1995).

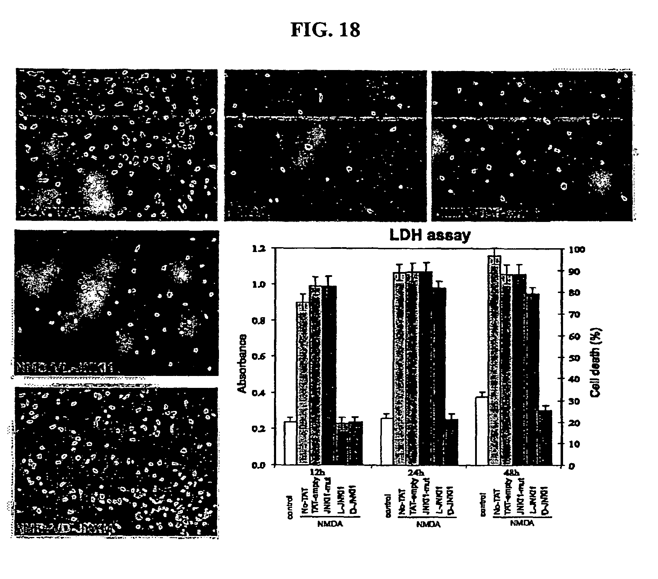

The time course of NMDA neurotoxicity and neuroprotection by L-JNKI1 and D-JNKI1, as well as two control peptides, TAT-empty (*i.e*., the TAT sequence alone, without the JBD₂₀ sequence) and L-JNKI1ₘᵤₜ (having six amino acids mutated to alanine, as described in Bonny et al., Diabetes 50:77-82 (2001) ). The micrographs of FIG. 18 show Hoechst-stained neurons at 24 hours after treatment. Addition of the L-JNKI and D-JNKI1 peptides completely protected neurons against the excitotoxic effects of NMDA (FIG.18) or kainate (data not shown), while the addition of control peptides had no neuroprotective effect. At 12 hours post-treatment, both L-JNKI1 and D-JNKI1 peptides were shown to inhibit neuronal death whereas TAT-empty peptides had no effect (FIG. 18).

As seen in FIG 18, the D-form of the cell-penetrating peptides of the invention, *i.e.*, D-JNKI1, was superior in protecting neurons for extended periods of time, *i.e*., 12 hours, 24 hours and 48 hours post-exposure to 100 µM NMDA. These micrographs indicate that at 24 hours post-treatment, D-JNKI1 still gave total neuroprotection, as the control cultures and the cultures treating with D-JNKI1 and NMDA were comparable. The L-form of JNKI1 no longer protected the neurons at 24 hours post-treatment, presumably because the L-forms of peptides are generally more susceptible to degradation. The TAT-empty peptides did not affect cell death in any conditions. The histogram in FIG. 18 depicts the level of neuronal death at 12, 24 and 48 hours after exposure to 100 µM NMDA, as indicated by LDH activity in the medium of the Petri dish. Absorbance values, which represent the LDH concentration, have been converted into % neuronal death values by dividing the absorbance values by the average absorbance for total LDH. The average absorbance for total LDH was obtained from the medium plus lysed neurons.

### Example 21: In Vivo delivery of cell-permeable JNKI peptides

To test the feasibility of using the cell-permeable peptides in *in vivo* applications, their ability to penetrate into the brain was evaluated using FITC-labeled L-JNKI1 and D-JNKI1. For a discussion on the *in vivo* delivery of a biologically active protein into a mouse, see Schwarze et al., Science, 285:1569-72 (1999) . These experiments showed that both FITC-labeled L-JNKI1 and D-JNKI1 were able to cross the blood-brain barrier and penetrate into the neurons of adult mice and rats of various ages. Both FTTC-labeled L-JNKI1 and D-JNKI1 were able to penetrate into the neurons within 1 hour of intraperitoneal injection (data not shown).

### Example 22: Neuroprotection by the JNKI peptides against transient and permanent focal cerebral ischemia

In a model of mild ischemia in mice, the left middle cerebral artery was occluded for 30 minutes, followed by 48 h of reperfusion. The control vehicle-treated group received an injection of phosphate buffer saline (PBS) only. In the control vehicle-treated group, this occlusion resulted systematically in a major infarction containing severely pyknotic cells, which were predominantly found in the cortex and the stratum in all brains, and in 7 of the brains, these cells were also found in the hippocampus. The mean infarction volume was 67.4 mm³ (n=12) in those subjects in the control vehicle-treated group.

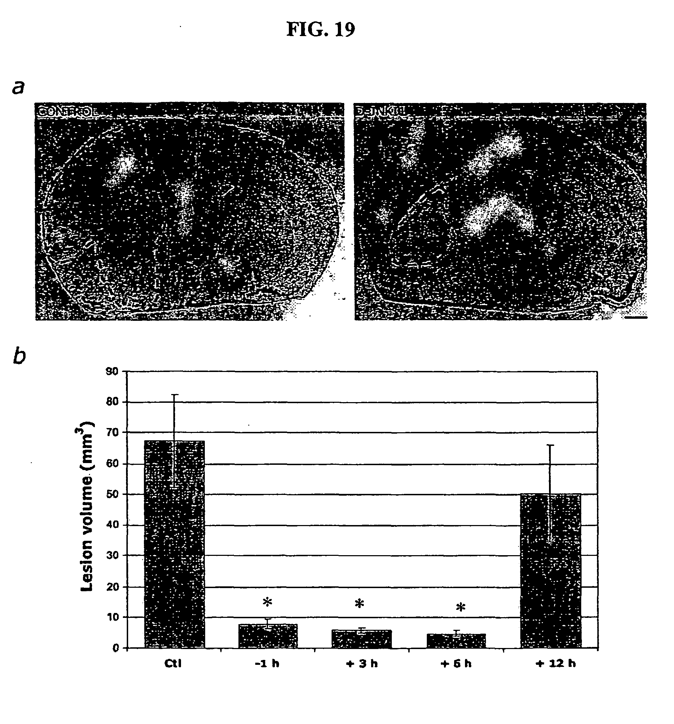

To evaluation the efficacy and "therapeutic window" of treatment (*i.e*., the timeframe following injury during which treatment with the peptides of the invention remains effective), subjects were treated with intracerebro-ventricular (icv) injection of D-JNKI1 (15.7 ng in 2 µL of PBS). FIG. 19A demonstrates cresyl violet-stained sections that show typical examples of the resulting infarct (bar, 1 mm). FIG. 19B depicts infarction volumes following icv injection of D-JNKI1 at different times before (-1 hour) or after (+3,6, or 12 hours) after middle cerebral artery occlusion. In FIG. 19B, an asterisk (*) indicates the result is statistically different from the control (as indicated by a t-test).

Pretreatment 1 hour before middle cerebral artery occlusion with the icv injection of D-JNKI1 significantly decreased the infarct volume measured 48 hours after reperfusion by 88%, to a volume of 7.8 mm³. (FIG. 19A-19B). Administering the D-JNKI1 peptide 3 or 6 hours after middle cerebral artery occlusion was still potently protective, as the mean infarct volume for subjects injected 3 hours post-occlusion was reduced to 5.8 mm³ (a reduction of 91% compared to untreated animals), and the mean infarct volume for subjects injected 6 hours post-occlusion was reduced to 4.8 mm³ (a reduction of 93% compared to untreated animals). In contrast, D-JNKI1 peptide injection at 12 hours after middle cerebral artery occlusion was not significantly protective. To confirm the achievement of complete ischemia followed by reperfusion was confirmed in all animals by monitoring regional cerebral blood flow in the territory of the left middle cerebral artery.

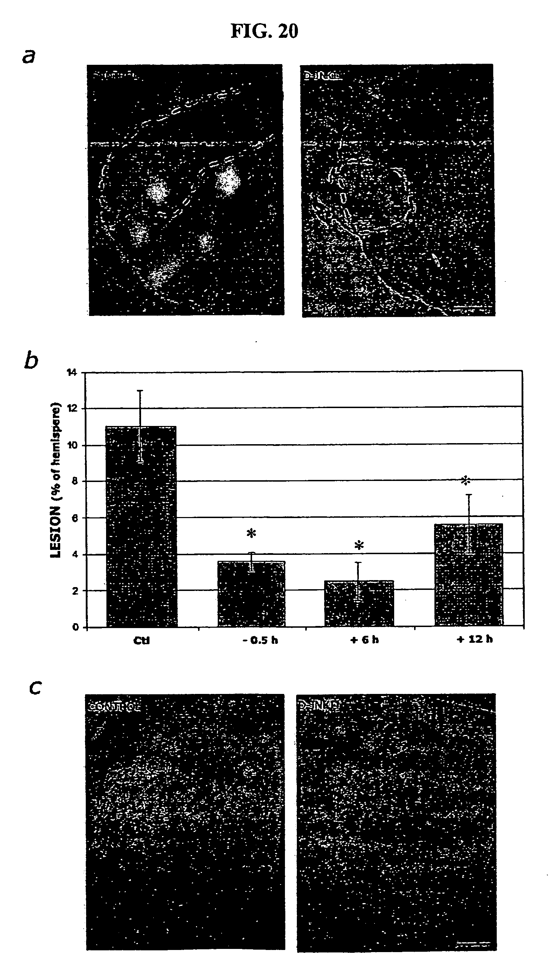

The protective abilities of D-JNKI1 against permanent focal ischemia in young (P14) rats was also evaluated. An ischemic zone in the cerebral cortex of P14 rats by performing a permanent occlusion of the middle cerebral artery, thereby inducing a zone of massive degeneration restricted to the parietotemporal cortex. As brain volumes in the P14 rats were variable, the lesions were expressed as a percentage of the volume of the cerebral hemisphere. D-JNKI1 was injected intraperitoneally at a concentration of 11 mg/kg, which corresponds to approximately 340 µg. D-JNKI1 was administered 30 minutes prior to middle cerebral artery occlusion, or 6 or 12 hours post-occlusion. The rats were fixed at 24 hours post-occlusion. At each of these time-points (*i.e*., administration at-30 minutes, + 6h or+12h), D-JNKI1 caused major and statistically significant decreases in the infarct volume, as compared to control animals (FIGS. 20A-20B). Administration of D-JNKI1 30 minutes prior to occlusion led to a decrease in the infarct volume of 68%, while peptide administration at 6 and 12 hours post-occlusion led to decreases in infarct volume of 78% and 49%, respectively.

Immunohistochemistry analysis was performed to determine the activation of the c-Jun transcription factor, a major target of JNK, in the brains of rat pups with permanent ischemia. Phosphorylation of c-Jun was evident in many neurons in the peri-infarcted cortex (FIG. 5C, bar = 200 µM). In contrast, in brains treated with D-JNKI1 peptide, the peri-infarcted cortex was negative, and only a few positive neurons at the border of the infarcted region were detected.

### Example 22: Behavioral evaluation of potential side-effects of JNKI peptides

Typically, the high toxicity of other neuroprotective compounds has severely limited their clinical use. (*See* Gladstone et al., Stroke, 33:2123-36 (2002) ). The ability of mice to maintain themselves on horizontal turning rotarod was used as criterion for possible side effects of different doses of D-JNKI1 and of a therapeutic dose of MK-801 (1 mg/Kg, a standard therapeutic dose). In particular, the motor function of the mice was evaluated using the rotarod test at 3h, 24h, 6 days and 12 days after both i.p. (11 and 110 mg/Kg) and icv injections of D-JNKI1 (2 µl containing 15.7 ng or 157 ng of D-JNKI1). The i.p. injection of MK-801 (1 mg/Kg) was used as a control compound during this assessment procedure.

The mice were trained the day before and in the morning of the experimental day, in order to reduce the variability between subjects. Both training and test sessions were identical for control and injected mice. The motor function of each mouse was examined immediately before the injection and at 1, 6 and 12 days after the injection. The mice were placed on the rotarod, which was programmed to accelerate uniformly from 4 to 40 rpm. The latency to falls for each mouse tested was recorded. The results of this assessment using the rotarod methods are presented in Table 2 as median latency to fall (measured in seconds).

**TABLE 2: EFFECT OF D-JNKI1 ON MOTOR COORDINATION**

| **Median latency to fall (sees)** | | | | | | |
|---|---|---|---|---|---|---|
| | **DOSE** | **-1 h** | **+3 h** | **1 day** | **6 days** | **12 days** |
| **PBS** | 2 µl icv | 234 | 202 | 238 | 268 | 246 |
| **MK-801** | 1 mg/Kg i.p. | 226 | incapable | 174 | 233 | 292 |
| **D-JNKI1** | 11 mg/Kg i.p. | 204 | 221 | 372 | 287 | 418 |
| | 110 mg/Kg i.p. | 276 | 266 | 447 | 416 | 325 |
| | 15.7 ng icv | 210 | 342 | 302 | 345 | 285 |
| | 157 ng icv | 260 | 200 | 253 | 338 | 335 |
| | 2 µl PBS icv | 234 | 202 | 238 | 268 | 246 |

As seen in Table 2, motor coordination was found to be unimpaired with both the i.p. and icv D-JNKI1 doses (*i.e.*, both the dose, 2.8 µl/Kg, that conferred 90% neuroprotection, and a 10-fold higher dose). In contrast. MK-801 led to a dramatic impairment of motor coordination, as the mice were unable to stand on the rotor wheel. (*See e.g.*, Table 2; Dawson et al., Brain Res. 892:344:350 (2001) (describing similar results for other neuroprotectants), and a 10-fold higher dose of MK-801 killed all the mice. The side effects of the lower dose of MK-801 were found to essentially disappear after 24 hours. At 6 and 15 days following treatment with D-JNKI1, no sign of motor impairment was found, and the rotarod scores were reproducibly better than in the control mice.

## Claims

1. The use of any one of the peptides including any one of the amino acid sequences selected from SEQ ID NOs: 3-6 and 22, or being any one of the amino acid sequences selected from SEQ ID NOs: 11-16 and 23-26, or of peptides being at least 95% homologous to these peptides, in the manufacture of a medicament for the treatment or prevention of hearing loss in a subject, wherein the peptide prevents damage to the hair cell stereocilia, hair cell apoptosis or neuronal apoptosis.

2. The use of a nucleic acid encoding any one of the peptides having L-amino acids and including the amino acid sequence of SEQ ID NO: 5, or being any one of the amino acid sequences selected from SEQ ID NOs: 11-13, 23 and 25; or encoding peptides being at least 95% homologous to these peptides, in the manufacture of a medicament for the treatment or prevention of hearing loss in a subject, wherein the peptide prevents damage to the hair cell stereocilia, hair cell apoptosis or neuronal apoptosis.

3. The use according to claim 1 or 2, wherein the peptide or nucleic acid used for the manufacture of a medicament for the treatment or prevention of hearing loss is to be administered before the subject is exposed to a noise trauma.

4. The use according to claim 1 or 2, wherein the peptide or nucleic acid used for the manufacture of a medicament for the treatment or prevention of hearing loss is administered after the subject is exposed to a noise trauma.

5. The use according to claim 3 or 4, wherein the noise trauma is a noise of at least 90 dB SPL.

6. The use according to claim 1 or 2, wherein the peptide or nucleic acid used for the manufacture of a medicament for the treatment or prevention of hearing loss is administered before the subject is exposed to an antibiotic.

7. The use according to claim 1 or 2, wherein the peptide or nucleic acid used for the manufacture of a medicament for the treatment or prevention of hearing loss is administered after the subject is exposed to an antibiotic.

8. The use according to claim 6 or 7, wherein the antibiotic is an aminoglycoside.

9. The use according to claim 1 or 2, wherein the peptide or nucleic acid used for the manufacture of a medicament for the treatment or prevention of hearing loss is administered before the subject is exposed to a chemotherapeutic agent.

10. The use according to claim 1 or 2, wherein the peptide or nucleic acid used for the manufacture of a medicament for the treatment or prevention of hearing loss is administered after the subject is exposed to a chemotherapeutic agent.

11. The use according to any one of claims 3 to 10, wherein the peptide or nucleic acid is administered by any one administration route selected from the group consisting of intrauricular, intraperitoneal, nasal, intravenous, oral and patch delivery.

## Patentansprüche

1. Verwendung von irgendeinem der Peptide, die jede beliebige der Aminosäuresequenzen von SEQ ID NO 3-6 und 22 einschließen oder jeder beliebigen der Aminosäuresequenzen von SEQ ID NO 11-16 und 23-26 entsprechen oder von Peptiden, die mindestens 95% homolog mit diesen Peptiden sind, zur Herstellung eines Medikaments zur Behandlung oder Vorbeugung von Hörverlust in einem Subjekt, wobei das Peptid eine Schädigung an den Haarzell-Stereozilien, Haarzell-Apoptose oder neuronale Apoptose verhindert.

2. Verwendung einer Nukleinsäure, die für irgendeines der Peptide, die L-Aminosäuren aufweisen und die die Aminosäuresequenz von SEQ ID NO 5 einschließen oder die jeder beliebigen der Aminosäuresequenzen von SEQ ID NO 11-13, 23 und 25 entsprechen, oder die für Peptide kodieren, die mindestens 95% homolog zu diesen Peptiden sind, zur Herstellung eines Medikaments zur Behandlung oder Vorbeugung von Hörverlust in einem Subjekt, wobei das Peptid eine Schädigung der Haarzell-Stereozilien, eine Haarzell-Apoptose oder neuronale Apoptose verhindert.

3. Verwendung nach Anspruch 1 oder 2, wobei das Peptid- oder die Nukleinsäure, die zur Herstellung eines Medikaments zur Behandlung oder Vorbeugung von Hörverlust verwendet werden, dem Subjekt verabreicht werden, bevor es einem Knalltrauma ausgesetzt wird.

4. Verwendung nach Anspruch 1 oder 2, wobei das Peptid oder die Nukleinsäure, die für die Herstellung eines Medikaments zur Behandlung oder zur Vorbeugung von Hörverlust verwendet werden, dem Subjekt verabreicht werden, nachdem es einem Knalltrauma ausgesetzt worden ist.

5. Verwendung nach Anspruch 3 oder 4, wobei das Knalltrauma einer Lautstärke von mindestens 90 dB SPL entspricht.

6. Verwendung nach Anspruch 1 oder 2, wobei das Peptid oder die Nukleinsäure, die zur Herstellung eines Medikaments zur Behandlung oder Vorbeugung von Hörverlust verwendet werden, dem Subjekt verabreicht werden, bevor dieses einem Antibiotikum ausgesetzt wird.

7. Verwendung nach Anspruch 1 oder 2, wobei das Peptid oder die Nukleinsäure, die zur Herstellung eines Medikaments zur Behandlung oder Vorbeugung von Hörverlust verwendet werden, dem Subjekt verabreicht werden, nachdem es einem Antibiotikum ausgesetzt worden ist.

8. Verwendung nach Anspruch 6 oder 7, wobei das Antibiotikum Aminoglykosid ist.

9. Verwendung nach Anspruch 1 oder 2, wobei das Peptid oder die Nukleinsäure, die zur Herstellung eines Medikaments zur Behandlung oder Vorbeugung von Hörverlust verwendet werden, dem Subjekt verabreicht werden, bevor es einem chemotherapeutischen Agens ausgesetzt wird.

10. Verwendung nach Anspruch 1 oder 2, wobei das Peptid oder die Nukleinsäure, die zur Herstellung eines Medikaments zur Behandlung oder Vorbeugung von Hörverlust verwendet werden, dem Subjekt verabreicht werden, nachdem es einem chemotherapeutischen Agens ausgesetzt worden ist.

11. Verwendung nach einem der Ansprüche 3 bis 10, wobei das Peptid oder die Nukleinsäure auf einem Verabreichungsweg verabreicht werden, ausgewählt aus der Gruppe, bestehend aus intrauricularer, intraperitonealer, nasaler, intravenöser, oraler Verabreichung und "patch delivery" (Patchabgabe).

## Revendications

1. Utilisation de l'un quelconque des peptides incluant un quelconque des séquences d'acides aminés choisie de SEQ ID n° 3 à 6 et 22, ou étant un quelconque des séquences d'acides aminés choisie de SEQ ID n° 11 à 16 et 23 à 26, ou des peptides étant au moins homologues à 95 % à ces peptides, pour la fabrication d'un médicament destiné au traitement ou à la prévention de la perte auditive chez un sujet, dans lequel le peptide empêche l'endommagement du stéréocil des cellules auditives, l'apoptose de cellules auditives ou l'apoptose neuronale.

2. Utilisation d'un acide nucléique codant l'un quelconque des peptides ayant des acides L-aminés et incluant la séquence d'acides aminés SEQ ID n° 5 ; ou étant un quelconque des séquences d'acides aminés choisie de SEQ ID n° 11 à 13, 23 et 25, ou codant des peptides étant au moins homologues à 95 % à ces peptides, pour la fabrication d'un médicament destiné au traitement ou à la prévention de la perte auditive chez un sujet, dans lequel le peptide empêche l'endommagement du stéréocil des cellules auditives, l'apoptose de cellules auditives ou l'apoptose neuronale.

3. Utilisation selon la revendication 1 ou 2, dans laquelle le peptide ou l'acide nucléique utilisé pour la fabrication d'un médicament destiné au traitement ou à la prévention de la perte auditive est administré avant l'exposition du sujet à un traumatisme sonore.

4. Utilisation selon la revendication 1 ou 2, dans laquelle le peptide ou l'acide nucléique utilisé pour la fabrication d'un médicament destiné au traitement ou à la prévention de la perte auditive est administré après l'exposition du sujet à un traumatisme sonore.

5. Utilisation selon la revendication 3 ou 4, dans laquelle le traumatisme sonore est un bruit d'au moins 90 dB SPL.

6. Utilisation selon la revendication 1 ou 2, dans laquelle le peptide ou l'acide nucléique utilisé pour la fabrication d'un médicament destiné au traitement ou à la prévention de la perte auditive est administré avant l'exposition du sujet à un antibiotique.

7. Utilisation selon la revendication 1 ou 2, dans laquelle le peptide ou l'acide nucléique utilisé pour la fabrication d'un médicament destiné au traitement ou à la prévention de la perte auditive est administré après l'exposition du sujet à un antibiotique.

8. Utilisation selon la revendication 6 ou 7, dans laquelle l'antibiotique est un aminoglycoside.

9. Utilisation selon la revendication 1 ou 2, dans laquelle le peptide ou l'acide nucléique utilisé pour la fabrication d'un médicament destiné au traitement ou à la prévention de la perte auditive est administré avant l'exposition du sujet à un agent chimiothérapeutique.

10. Utilisation selon la revendication 1 ou 2, dans laquelle le peptide ou l'acide nucléique utilisé pour la fabrication d'un médicament destiné au traitement ou à la prévention de la perte auditive est administré après l'exposition du sujet à un agent chimiothérapeutique.

11. Utilisation selon l'une quelconque des revendications 3 à 10, dans laquelle le peptide ou l'acide nucléique est administré par n'importe quel mode d'administration choisi dans le groupe constitué par les délivrances intraauriculaire, intrapéritonéale, nasale, intraveineuse, orale et au moyen d'un patch.


### SEQUENCE LISTING

<110> Xiagen S.A
<120> Cell-permeable peptide inhibitors of the JNK signal transduction pathway
<130> UO01P005WOEPT1
<140> EP 06019172
   <141> 2006-09-13
<150> US 10/165,250
   <151> 2002-06-07
<160> 33
<170> PatentIn version 3.3
<210> 1
   <211> 23
   <212> PRT
   <213> Unknown
<220>
   <223> Description of sequence: JNK inhibitor peptide L-IB1
<400> 1
   Asp Thr Tyr Arg Pro Lys Arg Pro Thr Thr Leu Asn Leu Phe Pro Gln
   1               5                   10                  15
   Val Pro Arg Ser Gln Asp Thr
               20
<210> 2.
   <211> 21
   <212> PRT
   <213> Unknown
<220>
   <223> Description of sequence: JNK inhibitor peptide L-IB2
<400> 2
   Glu Glu Pro His Lys His Arg Pro Thr Thr Leu Arg Leu Thr Thr Leu
   1               5                   10                  15
   Gly Ala Gln Asp Ser
               20
<210> 3
   <211> 23
   <212> PRT
   <213> Unknown
<220>
   <223> Description of sequence: JNK inhibitor peptide D-IB1
<400> 3
   Thr Asp Gln Ser Arg Pro Val Gln Pro Phe Leu Asn Leu Thr Thr Pro
   1 5 10 15
   Arg Lys Pro Arg Tyr Thr Asp
   20
<210> 4
   <211> 21
   <212> PRT
   <213> Unknown
<220>
   <223> Description of sequence: JNK inhibitor peptide D-IB2
<400> 4
   Ser Asp Gln Ala Gly Leu Thr Thr Leu Arg Leu Thr Thr Pro Arg His
   1 5 10 15
   Lys His Pro Glu Glu
   20
<210> 5
   <211> 19
   <212> PRT
   <213> Unknown
<220>
   <223> Description of sequence: JNK inhibitor peptide L-IB (generic) (XRPTTLXLXXXXXXXQDS/TX)
<220>
   <221> VARIATION
   <222> (1)..(1)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replace=""
<220>
   <221> VARIATION
   <222> (7)..(7)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replace=""
<220>
   <221> VARIATION
   <222> (9)..(15)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /-replace="Val"
   /replace="Xaa"
   /replace=""
<220>
   <221> VARIATION
   <222> (18)..(18)
   <223> /replace="Thr"
<220>
   <221> VARIATION
   <222> (19)..(19)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replace=""
<400> 5
   Ala Arg Pro Thr Thr Leu Ala Leu Ala Ala Ala Ala Ala Ala Ala Gln
   1               5                   10                  15
   Asp Ser Ala
<210> 6
   <211> 19
   <212> PRT
   <213> Unknown
<220>
   <223> Description of sequence: JNK inhibitor peptide D-IB (generic) (XS/TDQXXXXXXXLXLTTPRX)
<220>
   <221> VARIATION
   <222> (1)..(1)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replace=""
<220>
   <221> VARIATION
   <222> (2)..(2)
   <223> /replace="Thr"
<220>
   <221> VARIATION
   <222> (5)..(11)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replaced=""
<220>
   <221> VARIATION
   <222> (13)..(13)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replace=""
<220>
   <221> VARIATION
   <222> (19)..(19)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replace=""
<400> 6
   Ala Ser Asp Gln Ala Ala Ala Ala Ala Ala Ala Leu Ala Leu Thr Thr
   1 5 10 15
   Pro Arg Ala
<210> 7
   <211> 10
   <212> PRT
   <213> Unknown
<220>
   <223> Description of sequence: peptide containing TAT sequence L-TAT
<400> 7
   Gly Arg Lys Lys Arg Arg Gln Arg Arg Arg
   1 5 10
<210> 8
   <211> 10
   <212> PRT
   <213> Unknown
<220>
   <223> Description of sequence: retro-inverso peptide containing TAT sequence D-TAT
<400> 8
   Arg Arg Arg Gln Arg Arg Lys Lys Arg Gly
   1 5 10
<210> 9
   <211> 17
   <212> PRT
   <213> Unknown
<220>
   <223> Description of sequence: peptide containing generic TAT sequence L-generic-TAT
<220>
   <221> VARIATION
   <222> (1)..(4)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replace=""
<220>
   <221> VARIATION
   <222> (14)..(17)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replace=""
<400> 9
   Ala Ala Ala Ala Arg Lys Lys Arg Arg Gln Arg Arg Arg Ala Ala Ala
   1               5                   10                  15
   Ala
<210> 10
   <211> 17
   <212> PRT
   <213> Unknown
<220>
   <223> Description of sequence: peptide containing generic TAT sequence D-generic-TAT
<220>
   <221> VARIATION
   <222> (1)..(4)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replace=""
<220>
   <221> VARIATION
   <222> (14)..(17)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replace=""
<400> 10
   Ala Ala Ala Ala Arg Arg Arg Gln Arg Arg Lys Lys Arg Ala Ala Ala
   1               5                   10                  15
   Ala
<210> 11
   <211> 35
   <212> PRT
   <213> Unknown
<220>
   <223> Description of sequence: JNK inhibitor fusion protein L-TAT-IB1
<400> 11
   Gly Arg Lys Lys Arg Arg Gln Arg Arg Arg Pro Pro Asp Thr Tyr Arg
   1               5                   10                  15
   Pro Lys Arg Pro Thr Thr Leu Asn Leu Phe Pro Gln Val Pro Arg Ser
               20                  25                  30
   Gln Asp Thr
           35
<210> 12
   <211> 33
   <212> PRT
   <213> Unknown
<220>
   <223> Description of sequence: JNK inhibitor fusion protein L-TAT-IB2
<400> 12
   Gly Arg Lys Lys Arg Arg Gln Arg Arg Arg Pro Pro Glu Glu Pro His
   1               5                   10                  15
   Lys His Arg Pro Thr Thr Leu Arg Leu Thr Thr Leu Gly Ala Gln Asp
               20                  25                  30
   Ser
<210> 13
   <211> 42
   <212> PRT
   <213> Unknown
<220>
   <223> Description of sequence: JNK inhibitor fusion protein L-TAT-IB (generic) (XXXXXXXRKKRRQRRRXXXXXXXXRPTTLXLXXXXXXXQDS/TX)
<220>
   <221> VARIATION
   <222> (1)..(7)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replace=""
<220>
   <221> VARIATION
   <222> (17)..(24)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replace=""
<220>
   <221> VARIATION
   <222> (30)..(30)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replace=""
<220>
   <221> VARIATION
   <222> (32)..(38)
   <223> /=eplace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replace=""
<220>
   <221> VARIATION
   <222> (41)..(41)
   <223> /replace="Thr"
<220>
   <221> VARIATION
   <222> (42)..(42)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replace=""
<900> 13
<210> 14
   <211> 35
   <212> PRT
   <213> Unknown
<220>
   <223> Description of sequence: JNK inhibitor fusion protein D-TAT-IB1
<400> 14
   Thr Asp Gln Ser Arg Pro Val Gln Pro Phe Leu Asn Leu Thr Thr Pro
   1 5 10 15
   Arg Lys Pro Arg Tyr Thr Asp Pro Pro Arg Arg Arg Gln Arg Arg Lys
   20 25 30
   Lys Arg Gly
   35
<210> 15
   <211> 33
   <212> PRT
   <213> Unknown
<220>
   <223> Description of sequence: JNK inhibitor fusion protein D-TAT-IB2
<400> 15
   Ser Asp Gln Ala Gly Leu Thr Thr Leu Arg Leu Thr Thr Pro Arg His
   1               5                   10                  15
   Lys His Pro Glu Glu Pro Pro Arg Arg Arg Gln Arg Arg Lys Lys Arg
               20                  25                  30
   Gly
<210> 16
   <211> 42
   <212> PRT
   <213> Unknown
<220>
   <223> Description of sequence: JNK inhibitor fusion protein D-TAT-IB (generic) (XT/SDQXXXXXXXLXLTTPRXXXXXXXXRRRQRRKKRXXXXXXX)
<220>
   <221> VARIATION
   <222> (1)..(1)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replace=""
<220>
   <221> VARIATION
   <222> (2)..(2)
   <223> /replace="Ser"
<220>
   <221> VARIATION
   <222> (5)..(11)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replace=""
<220>
   <221> VARIATION
   <222> (13)..(13)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replace=""
<220>
   <221> VARIATION
   <222> (19)..(26)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met."
   /replace="Phe"
   /replace="Pro"
   /replace="Ser"
   /replace="Thr"
   /replace="Trp"
   /replace="Tyr"
   /replace="Val"
   /replace="Xaa" /replace=""
<220>
   <221> VARIATION
   <222> (36)..(92)
   <223> /replace="Arg"
   /replace="Asx"
   /replace="Cys"
   /replace="Glx" /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replace=""
<400> 16
   Ala Thr Asp Gln Ala Ala Ala Ala Ala Ala Ala Leu Ala Leu Thr Thr
   1               5                   10                  15
   Pro Arg Ala Ala Ala Ala Ala Ala Ala Ala Arg Arg Arg Gln Arg Arg
               20                  25                  30
   Lys Lys Arg Ala Ala Ala Ala Ala Ala Ala
           35                  40
<210> 17
   <211> 29
   <212> PRT
   <213> Unknown
<220>
   <223> Description of sequence: JBD of IB1, IB1-long
<900> 17
<210> 18
   <211> 27
   <212> PRT
   <213> Unknown
<220>
   <223> Description of sequence: JBD of IB2, IB2-long
<400> 18
   Ile Pro Ser Pro Ser Val Glu Glu Pro His Lys His Arg Pro Thr Thr
   1 5 10 15
   Leu Arg Leu Thr Thr Leu Gly Ala Gln Asp Ser
   20 25
<210> 19
   <211> 29
   <212> PRT
   <213> Unknown
<220>
   <223> Description of sequence: JBD of c-Jun, c-Jun
<400> 19
   Gly Ala Tyr Gly Tyr Ser Asn Pro Lys Ile Leu Lys Gln Ser Met Thr
   1 5 10 15
   Leu Asn Leu Ala Asp Pro Val Gly Asn Leu Lys Pro His
   20 25
<210> 20
   <211> 29
   <212> PRT
   <213> Unknown
<220>
   <223> Description of sequence: JBD of ATF2, ATF2
<400> 20
   Thr Asn Glu Asp His Leu Ala Val His Lys His Lys His Glu Met Thr
   1 5 10 15
   Leu Lys Phe Gly Pro Ala Arg Asn Asp Ser Val Ile Val
   20 25
<210> 21
   <211> 20
   <212> PRT
   <213> Unknown
<220>
   <223> Description of sequence: JNK inhibitor peptide L-JBD20
<400> 21
   Arg Pro Lys Arg Pro Thr Thr Leu Asn Leu Phe Pro Gln Val Pro Arg
   1 5 10 15
   Ser Gln Asp Thr
   20
<210> 22
   <211> 20
   <212> PRT
   <213> Unknown
<220>
   <223> Description of sequence: JNK inhibitor peptide D-JBD20
<400> 22
   Thr Asp Gln Ser Arg Pro Val Gln Pro Phe Leu Asn Leu Thr Thr Pro
   1 5 10 15
   Arg Lys Pro Arg
   20
<210> 23
   <211> 32
   <212> PRT
   <213> Unknown
<220>
   <223> Description of sequence: JNK inhibitor fusion protein L-TAT-JNKI1 (i.e., L-TAT-JBD20)
<400> 23
   Gly Arg Lys Lys Arg Arg Gln Arg Arg Arg Pro Pro Arg Pro Lys Arg
   1 5 10 15
   Pro Thr Thr Leu Asn Leu Phe Pro Gln Val Pro Arg Ser Gln Asp Thr
   20 25 30
<210> 24
   <211> 32
   <212> PRT
   <213> Unknown
<220>
   <223> Description of sequence: JNK inhibitor fusion protein D-TAT-JNKI1 (i.e., D-TAT-JBD20)
<400> 24
   Thr Asp Gln Ser Arg Pro Val Gln Pro Phe Leu Asn Leu Thr Thr Pro
   1               5                   10                  15
   Arg Lys Pro Arg Pro Pro Arg Arg Arg Gln Arg Arg Lys Lys Arg Gly
               20                  25                  30
<210> 25
   <211> 34
   <212> PRT
   <213> Unknown
<220>
   <223> Description of sequence: JNK inhibitor fusion protein L-TAT-JNKI1 (generic) (XXXXRKKRRQRRRXXXXRPTTLXLXXXXXXXQDS/T)
<220>
   <221> VARIATION
   <222> (1)..(4)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa" /replace=""
<220>
   <221> VARIATION
   <222> (14)..(17)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa" /replace=""
<220>
   <221> VARIATION
   <222> (23)..(23)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replace=""
<220>
   <221> VARIATION
   <222> (25)..(31)
   <223> /replace="Arg"
   /replace="Asx"
   /replace="Cys"
   /replace="Glx"
   /replace="Gly"
   /replace="His"
   /replace="Ile"
   /replace="Leu"
   /,replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replace=""
<220>
   <221> VARIATION
   <222> (39)..(39)
   <223> /replace="Thr"
<400> 25
   Ala Ala Ala Ala Arg Lys Lys Arg Arg Gln Arg Arg Arg Ala Ala Ala
   1 5 10 15
   Ala Arg Pro Thr Thr Leu Ala Leu Ala Ala Ala Ala Ala Ala Ala Gln
   20 25 30
   Asp Ser
<210> 26
   <211> 34
   <212> PRT
   <213> Unknown
<220>
   <223> Description of sequence: JNK inhibitor fusion protein D-TAT-JNKI1 (generic) (S/TDQXXXXXXXLXLTTPRXXXXRRRQRRKKRXXXX)
<220>
   <221> VARIATION
   <222> (1)..(1)
   <223> /replace="Thr"
<220>
   <221> VARIATION
   <222> (4)..(10)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replace=""
<220>
   <221> VARIATION
   <222> (12)..(12)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replace=""
<220>
   <221> VARIATION
   <222> (18)..(21)
   <223> /replace="Arg"
   /replace="Asx"
   /replace="Cys"
   /replace="Glx"
   /replace="Gly"
   /replace="His"
   /replace="Ile"
   Ireplace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replace=""
<220>
   <221> VARIATION
   <222> (31)..(39)
   <223> /replace="Arg"
   /replace="Asx"
   /replace="Cys"
   /replace="Glx"
   /replace="Gly"
   /replace="His"
   /replace="Ile"
   /replace="Leu"
   /replace="Lys"
   /replace="Met"
   /replace="Phe"
   /replace="Pro"
   /replace="Ser"
   /replace="Thr"
   /replace="Trp"
   /replace="Tyr"
   /replace="Val"
   /replace="Xaa"
   /replace=""
<400> 26
   Ser Asp Gln Ala Ala Ala Ala Ala Ala Ala Leu Ala Leu Thr Thr Pro
   1 5 10 15
   Arg Ala Ala Ala Ala Arg Arg Arg Gln Arg Arg Lys Lys Arg Ala Ala
   20 25 30
   Ala Ala
<210> 27
   <211> 20
   <212> PRT
   <213> Unknown
<220>
   <223> Description of sequence: JNK inhibitor peptide L-JBD20-mut
<400> 27
   Arg Pro Lys Arg Pro Thr Ala Ala Asn Ala Phe Pro Gln Val Pro Arg
   1 5 10 15
   Ser Gln Asp Thr
   20
<210> 28
   <211> 20
   <212> PRT
   <213> Unknown
<220>
   <223> Description of sequence: JNK inhibitor peptide D-JBD20-mut
<400> 28
   Thr Asp Gln Ser Arg Pro Val Ala Pro Phe Ala Asn Ala Ala Thr Pro
   1 5 10 15
   Arg Lys Pro Arg
   20
<210> 29
   <211> 21
   <212> DNA
   <213> Unknown
<220>
   <223> Description of sequence: AP-1 doubled labeled probe (see page 45 of the description)
<400> 29
   cgcttgatga gtcagccgga a 21
<210> 30
   <211> 20
   <212> DNA
   <213> Unknown
<220>
   <223> Description of sequence: RT-PCR c-Fos primer forward (see page 48 of the description)
<400> 30
   gctgacagat acactccaag 20
<210> 31
   <211> 20
   <212> DNA
   <213> Unknown
<220>
   <223> Description of sequence: RT-PCR c-Fos primer reverse (see page 48 of the description)
<400> 31
   cctagatgat gccggaaaca 20
<210> 32
   <211> 20
   <212> DNA
   <213> Unknown
<220>
   <223> Description of sequence: RT-PCR Actin primer forward (see page 48 of the description)
<400> 32
   aacggctccg gcatgtgcaa 20
<210> 33
   <211> 21
   <212> DNA
   <213> Unknown
<220>
   <223> Description of sequence: RT-PCR Actin primer reverse (see page 48 of the description)
<400> 33
   attgtagaag gtgtggtgcc a 21